# EUROPEAN PATENT APPLICATION

(11) **EP 4 079 855 A1**
(43) Date of publication of application: **26.10.2022**
(21) Application number: 21169352.8
(22) Date of filing: 20.04.2021
(51) Int. Cl.: C12N 15/113, C12N 9/22, A61K 31/136, A61K 31/7088, A61P 35/02

(54) **COMPOUND AND METHOD FOR THE PROPHYLAXIS AND TREATMENT OF LEUKEMIA**

(71) Applicant: Eberhard Karls Universität Tübingen Medizinische Fakultät, 72074 Tübingen (DE)
(72) Inventor: Skokowa, Julia, 72127 Kusteringen (DE); Dannenmann, Benjamin, 75387 Neubulach (DE); Klimiankou, Maksim, 72076 Tübingen (DE)
(74) Representative: Witte, Weller & Partner Patentanwälte mbB

(57) **Abstract**

The invention is directed to a compound for use in the prophylaxis and/or treatment of leukemia and/or the development of leukemia, a pharmaceutical composition for the prophylaxis and/or treatment of leukemia and/or the development of leukemia, comprising said compound, a method for the prophylaxis and/or treatment of leukemia and/or the development of leukemia in a living being, a single guide RNA (sgRNA) molecule which can be used in the method according to the invention.

SEQUENCE LISTING

<110>  Eberhard Karls Universität Tübingen - Medizinische Fakultät

<120>  Compound and method for the prophylaxis and treatment of leukemia

<130>  5402P627EP

<160>  78

<170>  PatentIn version 3.5

<210>  1
<211>  20
<212>  DNA
<213>  Artificial Sequence

<220>
<223>  None

<400>  1
gtccagctct cgtagtagcg                                                   20


<210>  2
<211>  20
<212>  DNA
<213>  Artificial Sequence

<220>
<223>  None

<400>  2
gggtccagct ctcgtagtag                                                   20


<210>  3
<211>  20
<212>  DNA
<213>  Artificial Sequence

<220>
<223>  None

<400>  3
catcttgcct gggctcagcg                                                   20


<210>  4
<211>  20
<212>  DNA
<213>  Artificial Sequence

<220>
<223>  None

<400>  4
cacttacttc gaggttctcg                                                   20


<210>  5
<211>  20
<212>  DNA

<213>  Artificial Sequence

<220>
<223>  None

<400>  5
ctggtgccag actgggattg                                                    20


<210>  6
<211>  20
<212>  DNA
<213>  Artificial Sequence

<220>
<223>  None

<400>  6
gctgggtgga aactgctctt                                                    20


<210>  7
<211>  20
<212>  DNA
<213>  Artificial Sequence

<220>
<223>  None

<400>  7
gcgcggcggt gcacacgcag                                                    20


<210>  8
<211>  20
<212>  DNA
<213>  Artificial Sequence

<220>
<223>  None

<400>  8
agtgagtttg tagtttggca                                                    20


<210>  9
<211>  23
<212>  DNA
<213>  Artificial Sequence

<220>
<223>  None

<400>  9
ctccagccct cacagtagcg agg                                                23


<210>  10
<211>  23
<212>  DNA
<213>  Artificial Sequence

<220>

<223>  None

<400>  10
ggccagctct agtggcagcg cgg                                               23


<210>  11
<211>  23
<212>  DNA
<213>  Artificial Sequence

<220>
<223>  None

<400>  11
ttcctgctct cttagtagct agg                                               23


<210>  12
<211>  23
<212>  DNA
<213>  Artificial Sequence

<220>
<223>  None

<400>  12
gggtccagct atcttactag tgg                                               23


<210>  13
<211>  23
<212>  DNA
<213>  Artificial Sequence

<220>
<223>  None

<400>  13
gtttccagct ctagtagtag tag                                               23


<210>  14
<211>  23
<212>  DNA
<213>  Artificial Sequence

<220>
<223>  None

<400>  14
gggtccagct ctccttggag agg                                               23


<210>  15
<211>  23
<212>  DNA
<213>  Artificial Sequence

<220>
<223>  None

<400>  15

catcctgcct gggctcagct ggg                                                23

<210>  16
<211>  23
<212>  DNA
<213>  Artificial Sequence

<220>
<223>  None

<400>  16
   catttgccca aggctcagcg tgg                                                23

<210>  17
<211>  23
<212>  DNA
<213>  Artificial Sequence

<220>
<223>  None

<400>  17
gatcttgtct gggctcagct ggg                                                23

<210>  18
<211>  23
<212>  DNA
<213>  Artificial Sequence

<220>
<223>  None

<400>  18
tatttactct gaggttctcg cgg                                                23

<210>  19
<211>  23
<212>  DNA
<213>  Artificial Sequence

<220>
<223>  None

<400>  19
cacttactat gtagttctcg tgg                                                23

<210>  20
<211>  23
<212>  DNA
<213>  Artificial Sequence

<220>
<223>  None

<400>  20
tacttacttc tgggttcccg agg                                                23

<210>  21
<211>  23
<212>  DNA
<213>  Artificial Sequence

<220>
<223>  None

<400>  21
cagttgccag actgggattg agg                                               23


<210>  22
<211>  23
<212>  DNA
<213>  Artificial Sequence

<220>
<223>  None

<400>  22
ctggcgcctg actaggattg agg                                               23


<210>  23
<211>  23
<212>  DNA
<213>  Artificial Sequence

<220>
<223>  None

<400>  23
caggtgaaag actgggattg aag                                               23


<210>  24
<211>  23
<212>  DNA
<213>  Artificial Sequence

<220>
<223>  None

<400>  24
gctgggtgta agctgccctt agg                                               23


<210>  25
<211>  23
<212>  DNA
<213>  Artificial Sequence

<220>
<223>  None

<400>  25
gctgggagga aactgctcat gga                                               23


<210>  26
<211>  23
<212>  DNA

<213>  Artificial Sequence

<220>
<223>  None

<400>  26
gatggctgga aactgctcct ggg                                               23


<210>  27
<211>  23
<212>  DNA
<213>  Artificial Sequence

<220>
<223>  None

<400>  27
ctgcggggtt gcacacgcag cgg                                               23


<210>  28
<211>  23
<212>  DNA
<213>  Artificial Sequence

<220>
<223>  None

<400>  28
aggaggcagt gcacacgcag agg                                               23


<210>  29
<211>  23
<212>  DNA
<213>  Artificial Sequence

<220>
<223>  None

<400>  29
gcggggcggg gcacacacag ggg                                               23


<210>  30
<211>  23
<212>  DNA
<213>  Artificial Sequence

<220>
<223>  None

<400>  30
agtaagcctg tagttaggca tgg                                               23


<210>  31
<211>  23
<212>  DNA
<213>  Artificial Sequence

<220>

<223>  None

<400>  31
agtgagtatt tagtttggct ggg                                              23


<210>  32
<211>  23
<212>  DNA
<213>  Artificial Sequence

<220>
<223>  None

<400>  32
tgtgtgtttg tggtttggca ggg                                              23


<210>  33
<211>  25
<212>  DNA
<213>  Artificial Sequence

<220>
<223>  None

<400>  33
acatccctga tgtctgcatt tgtcc                                            25


<210>  34
<211>  25
<212>  DNA
<213>  Artificial Sequence

<220>
<223>  None

<400>  34
tgtgggtttg ttgccatgaa acgtg                                            25


<210>  35
<211>  20
<212>  DNA
<213>  Artificial Sequence

<220>
<223>  None

<400>  35
cgccctgagc cttggtgacg                                                  20


<210>  36
<211>  20
<212>  DNA
<213>  Artificial Sequence

<220>
<223>  None

<400>  36

agccacggtg cctgttgctg                                                    20

<210>  37
<211>  20
<212>  DNA
<213>  Artificial Sequence

<220>
<223>  None

<400>  37
atggcatgtg tcaggcatgt                                                    20

<210>  38
<211>  20
<212>  DNA
<213>  Artificial Sequence

<220>
<223>  None

<400>  38
agtcacagcg gagatagtgc                                                    20

<210>  39
<211>  20
<212>  DNA
<213>  Artificial Sequence

<220>
<223>  None

<400>  39
agccccgcta ctacgagagc                                                    20

<210>  40
<211>  20
<212>  DNA
<213>  Artificial Sequence

<220>
<223>  None

<400>  40
cggcgcgtcc gagtcggtgt                                                    20

<210>  41
<211>  20
<212>  DNA
<213>  Artificial Sequence

<220>
<223>  None

<400>  41
gctgggcggc gcgtccgagt                                                    20

<210>  42
<211>  20
<212>  DNA
<213>  Artificial Sequence

<220>
<223>  None

<400>  42
ctcgtagtag cggggctcga                                                   20


<210>  43
<211>  20
<212>  DNA
<213>  Artificial Sequence

<220>
<223>  None

<400>  43
gagtgcaggc cgcccgcttc                                                   20


<210>  44
<211>  20
<212>  DNA
<213>  Artificial Sequence

<220>
<223>  None

<400>  44
ctggctcacc tacaccgact                                                   20


<210>  45
<211>  20
<212>  DNA
<213>  Artificial Sequence

<220>
<223>  None

<400>  45
ccggccactt acccgagtgc                                                   20


<210>  46
<211>  20
<212>  DNA
<213>  Artificial Sequence

<220>
<223>  None

<400>  46
cgagtgcagg ccgcccgctt                                                   20


<210>  47
<211>  20
<212>  DNA

<213>  Artificial Sequence

<220>
<223>  None

<400>  47
gctactacga gagctggacc                                                   20


<210>  48
<211>  20
<212>  DNA
<213>  Artificial Sequence

<220>
<223>  None

<400>  48
ggtccagctc tcgtagtagc                                                   20


<210>  49
<211>  20
<212>  DNA
<213>  Artificial Sequence

<220>
<223>  None

<400>  49
ggggctcgat ggcatccgcc                                                   20


<210>  50
<211>  20
<212>  DNA
<213>  Artificial Sequence

<220>
<223>  None

<400>  50
agtgcaggcc gcccgcttcg                                                   20


<210>  51
<211>  20
<212>  DNA
<213>  Artificial Sequence

<220>
<223>  None

<400>  51
cgaagcgggc ggcctgcact                                                   20


<210>  52
<211>  20
<212>  DNA
<213>  Artificial Sequence

<220>

<223>  None

<400>  52
agcgccgccg ccccggacag                                                   20


<210>  53
<211>  20
<212>  DNA
<213>  Artificial Sequence

<220>
<223>  None

<400>  53
ctactacgag agctggaccc                                                   20


<210>  54
<211>  20
<212>  DNA
<213>  Artificial Sequence

<220>
<223>  None

<400>  54
cgagtcggtg taggtgagcc                                                   20


<210>  55
<211>  20
<212>  DNA
<213>  Artificial Sequence

<220>
<223>  None

<400>  55
cgcttcgggg ccgctgtccg                                                   20


<210>  56
<211>  20
<212>  DNA
<213>  Artificial Sequence

<220>
<223>  None

<400>  56
gaagcgggcg gcctgcactc                                                   20


<210>  57
<211>  20
<212>  DNA
<213>  Artificial Sequence

<220>
<223>  None

<400>  57

cccgcttcgg ggccgctgtc                                                    20

<210>  58
<211>  20
<212>  DNA
<213>  Artificial Sequence

<220>
<223>  None

<400>  58
ccggacagcg gccccgaagc                                                    20

<210>  59
<211>  20
<212>  DNA
<213>  Artificial Sequence

<220>
<223>  None

<400>  59
cccgggagac agaatccacc                                                    20

<210>  60
<211>  20
<212>  DNA
<213>  Artificial Sequence

<220>
<223>  None

<400>  60
cctgcactcg ggtaagtggc                                                    20

<210>  61
<211>  20
<212>  DNA
<213>  Artificial Sequence

<220>
<223>  None

<400>  61
ccgcttcggg gccgctgtcc                                                    20

<210>  62
<211>  20
<212>  DNA
<213>  Artificial Sequence

<220>
<223>  None

<400>  62
gcggcctgca ctcgggtaag                                                    20

<210>  63
<211>  20
<212>  DNA
<213>  Artificial Sequence

<220>
<223>  None

<400>  63
cccggacagc ggccccgaag                                                    20


<210>  64
<211>  20
<212>  DNA
<213>  Artificial Sequence

<220>
<223>  None

<400>  64
gacagcggcc ccgaagcggg                                                    20


<210>  65
<211>  20
<212>  DNA
<213>  Artificial Sequence

<220>
<223>  None

<400>  65
ttcggggccg ctgtccgggg                                                    20


<210>  66
<211>  20
<212>  DNA
<213>  Artificial Sequence

<220>
<223>  None

<400>  66
ctgtccgggg cggcggcgct                                                    20


<210>  67
<211>  20
<212>  DNA
<213>  Artificial Sequence

<220>
<223>  None

<400>  67
gtcggtgtag gtgagccagg                                                    20


<210>  68
<211>  20
<212>  DNA

<213>  Artificial Sequence

<220>
<223>  None

<400>  68
gctgtccggg gcggcggcgc                                                   20


<210>  69
<211>  20
<212>  DNA
<213>  Artificial Sequence

<220>
<223>  None

<400>  69
gccaggtgga ttctgtctcc                                                   20


<210>  70
<211>  20
<212>  DNA
<213>  Artificial Sequence

<220>
<223>  None

<400>  70
ccaggtggat tctgtctccc                                                   20


<210>  71
<211>  20
<212>  DNA
<213>  Artificial Sequence

<220>
<223>  None

<400>  71
gccgcccagc gccgccgccc                                                   20


<210>  72
<211>  20
<212>  DNA
<213>  Artificial Sequence

<220>
<223>  None

<400>  72
gatgggctgc ggcgggagcc                                                   20


<210>  73
<211>  20
<212>  DNA
<213>  Artificial Sequence

<220>

<223>  None

<400>  73
gcaggaggat gggctgcggc                                                    20


<210>  74
<211>  20
<212>  DNA
<213>  Artificial Sequence

<220>
<223>  None

<400>  74
tccgggcgg cggcgctggg                                                     20


<210>  75
<211>  20
<212>  DNA
<213>  Artificial Sequence

<220>
<223>  None

<400>  75
ggggccgctg tccgggcgg                                                     20


<210>  76
<211>  20
<212>  DNA
<213>  Artificial Sequence

<220>
<223>  None

<400>  76
cgcaggagga tgggctgcgg                                                    20


<210>  77
<211>  20
<212>  DNA
<213>  Artificial Sequence

<220>
<223>  None

<400>  77
ggatgggctg cggcgggagc                                                    20


<210>  78
<211>  20
<212>  DNA
<213>  Artificial Sequence

<220>
<223>  None

<400>  78

gggctgcggc gggagccggg 20

## Description

The invention is directed to a compound for use in the prophylaxis and/or treatment of leukemia and/or the development of leukemia, a pharmaceutical composition for the prophylaxis and/or treatment of leukemia and/or the development of leukemia, comprising said compound, a method for the prophylaxis and/or treatment of leukemia and/or the development of leukemia in a living being, a single guide RNA (sgRNA) molecule which can be used in the method according to the invention.

### FIELD OF THE INVENTION

The present invention relates to the field of molecular medicine, more particular to the field of small molecules and of genetic engineering applications, preferably to the targeted knockout of disease-associated genes.

### BACKGROUND OF THE INVENTION

Leukemia, also spelled leukaemia, is a group of blood cancers that usually begin in the bone marrow and result in high numbers of abnormal blood cells. These blood cells are not fully developed and are called blasts or leukemia cells. Symptoms may include bleeding and bruising, fatigue, fever, and an increased risk of infections. These symptoms occur due to a lack of normal blood cells. Diagnosis is typically made by blood tests or bone marrow biopsy.

The exact cause of leukemia is unknown. A combination of genetic factors and environmental (non-inherited) factors are believed to play a role. Risk factors include smoking, ionizing radiation, some chemicals (such as benzene), prior chemotherapy, and Down syndrome. People with a family history of leukemia are also at higher risk.

There are four main types of leukemia - acute lymphoblastic leukemia (ALL), acute myeloid leukemia (AML), chronic lymphocytic leukemia (CLL) and chronic myeloid leukemia (CML) - as well as a number of less common types. Leukemias and lymphomas both belong to a broader group of tumors that affect the blood, bone marrow, and lymphoid system, known as tumors of the hematopoietic and lymphoid tissues.

In 2015, leukemia was present in 2.3 million people worldwide and caused 353,500 deaths. In 2012 it newly developed in 352,000 people. It is the most common type of cancer in children, with three-quarters of leukemia cases in children being the acute lymphoblastic type. However, over 90% of all leukemias are diagnosed in adults, with CLL and AML being most common in adults. It occurs more commonly in the developed world.

Severe congenital neutropenia (CN) is a pre-leukemia syndrome that, in the majority of patients, is caused by heterogeneous *ELANE* mutations encoding neutrophil elastase (NE). The hematopoietic stem and progenitor cells (HSPCs) of CN patients fail to differentiate into neutrophilic granulocytes, without any severe maturation defects in other blood lineages. The cumulative incidence of MDS or AML in CN patients is approximately 20% after 20 years. Exposure of CN-HSPCs to high concentrations of granulocyte colony-stimulating factor (G-CSF) partially reverses granulocytic maturation defects. However, there is a correlation between susceptibility to G-CSF therapy and frequency of leukemia.

Treatment of leukemia may involve some combination of chemotherapy, radiation therapy, targeted therapy, and bone marrow transplant, in addition to supportive care and palliative care as needed. Certain types of leukemia may be managed with watchful waiting. The success of treatment depends on the type of leukemia and the age of the person. Outcomes have improved in the developed world. Five-year survival rate is 57% in the United States.

Against this background there is a need for novel therapeutic and prophylactic approaches which specifically and selectively interfere with the leukemogenesis, thereby not only allowing the healing or at least attenuation of an existing leukemia but also preventing the development thereof originating from a pre-leukemia status.

### SUMMARY OF THE INVENTION

This object is met by the provision of an inhibitor of 'mitogen-activated protein kinase-activated protein kinase 2' (MK2 inhibitor) for use in the prophylaxis and/or treatment of leukemia and/or the development of leukemia.

According to the invention "mitogen-activated protein kinase-activated protein kinase 2", also designated as MK2, MAPKAPK2, MAPKAP-K2, MK-2, mitogen-activated protein kinase-activated protein kinase 2, or MAPK activated protein kinase 2, refers to an enzyme that in humans is encoded by the MAPKAPK2 gene. This gene encodes a member of the Ser/Thr protein kinase family. This kinase is regulated through direct phosphorylation by p38 MAP kinase. In conjunction with p38 MAP kinase, this kinase is known to be involved in many cellular processes including stress and inflammatory responses, nuclear export, gene expression regulation and cell proliferation.

According to the invention "MK2 inhibitor" refers to a molecule which specifically inhibits the activity of MK2. In a preferred embodiment the MK2 inhibitor is a small molecule or an organic compound having a molecular size of < 900 daltons. Examples of MK2 inhibitors are CMPD1, PF 3644022, PHA 767491 hydrochloride, MK2-IN-1 hydrochloride, MK2-IN-3 hydrate, CDD-450, MK2 inhibitor IV (MK 25; CAS no: 1314118-94-9) etc. Alternatively, the MK2 inhibitor can be a peptide or a small protein.

According to the invention not only an existing leukemia can be effectively treated by the MK2 inhibitor. The inventors have also realized that the development of leukemia can be prevented by the use of an MK2 inhibitor. This is of particular importance because leukemia is often the result of a pre-existing condition such as pre-leukemia bone marrow failure syndromes e.g., congenital neutropenia (CN), familial platelet disorder with predisposition to acute myelogenous leukemia (FPD/AML) or myelodysplastic syndrome (MDS), secondary therapy-related leukemias or others. The invention also effectively remedies this situation and prevents the development of leukemia due to such pre-existing conditions. The invention, therefore, includes also the use of an MK2 inhibitor to prevent a relapsed leukemia, in particular relapsed AML.

In particular, according to the invention the MK2 inhibitor can be used to prevent or reduce the uncontrolled proliferation of AML blasts, in e.g. case of recurrence or in the period between diagnosis and bone marrow transplantation.

According to the invention, in an embodiment, the MK2 inhibitor can be used as the only active agent, i.e. in form of a monotherapeutics, without any additional further active agent. It is understood that the anti-leukemia activity according to the invention is exerted by the MK2 inhibitor. In an embodiment of the invention a synergistic activity on an other compound which is used against leukemia, e.g. SAHA (vorinostat), is explicitly excluded and not subject of the present invention.

The findings of the inventors were surprising and could not be expected.

In the art the use of MK2 inhibitors is described in relation with several tumors, such as glioblastoma (Gurgis et al., 2015, Cytotoxic activity of the MK2 inhibitor CMPD1 in glioblastoma cells is independent of MK2, Cell Death Discovery 1, 15028, p. 1-11), or gastric cancer (Li et al., 2018, CMPD1 inhibited human gastric cancer cell proliferation by inducing apoptosis and G2/M cell cycle arrest, Biol. Res. 51:11, p. 1-9).

Bogenberger et al. (2014), MK2a substrate-selective p38 inhibitor CMPD1 selectively synergizes with HDAC inhibitors in CD34+ cells from myeloid malignancies, Blood (2014) 124 (21): 2239, disclose the histone deacetylase inhibitor SAHA (vorinostat) as having a low clinical activity against myelodysplastic syndrome (MDS) and acute myeloid leukemia (AML). The authors propose to add CMPD1 to achieve a synergy. However, the authors were not able to demonstrate any activity of CMPD1 if administered alone. To the contrary, the authors even indicate that CMPD1 does not affect the regulation of several canonical MK2 targets at doses that are potently synergistic in AML cell lines, including the phosphorylation of HSP27 or AATF, and rather teach away from the invention.

The art is, therefore, silent about using an MK2 inhibitor for the prophylaxis or treatment of leukemia or the development thereof.

In another embodiment of the invention said leukemia is acute myeloid leukemia (AML).

This measure has the advantage that one of the most clinically relevant forms of leukemia is effectively addressed, which the art fails so far in doing so. This embodiment includes all different kinds of AML, such as *de novo* AML, RUNX1-mutated AML, secondary therapy-related AML etc.

In another embodiment of the invention said AML is *de novo* AML.

The inventors have surprisingly realized in an experimental set-up that for variants of AML which occur independently and without prior bone marrow disease or cancer, the administration of an MK2 inhibitor is of particular effectiveness.

In another embodiment of the invention said AML is AML with high expression of BAALC (BAALC^{high}).

According to the invention "BAALC" refers to a gene that codes for the 'brain and acute leukemia cytoplasmic' protein (human variant: NCBI gene no. 79870). The function of BAALC is not fully understood yet but high BAALC expression level is known to be associated with more aggressive AML and has been described to have adverse impact on the outcome of AML; see Baldus et al. (2003), BAALC expression predicts clinical outcome of de novo acute myeloid leukemia patients with normal cytogenetics: a Cancer and Leukemia Group B Study, Blood 102(5), p. 1613-1618; Baldus et al. (2006), BAALC expression and FLT3 internal tandem duplication mutations in acute myeloid leukemia patients with normal cytogenetics: prognostic implications, J. Clin. Oncol. 24(5), p. 790-797; Weber et al. (2014), BAALC expression: a suitable marker for prognostic risk stratification and detection of residual disease in cytogenetically normal acute myeloid leukemia, Blood Cancer J. 4(1):e173. This measure has the advantage that a particularly aggressive form of AML can be effectively treated and/or prevented by the invention.

In a further embodiment of the invention said development of leukemia results from a pre-existing condition that is selected from the group consisting of: congenital neutropenia (CN), familial platelet disorder with predisposition to acute myelogenous leukemia (FPD/AML) or myelodysplastic syndrome (MDS).

The indicated conditions are well-known for pre-existing diseases which are high risk factors for a subsequent development of AML. In this embodiment the invention can be effectively used in an early stage prior to or in the development of AML to eliminate or at least significantly reduce the risk of a progression of the pre-existing conditions. The invention can even be used for the prophylaxis and/or treatment of the indicated pre-conditions, thereby effectively preventing the manifestation of leukemia or AML, respectively.

In another embodiment of the invention the MK2 inhibitor is an MK2a inhibitor.

This measure has the advantage that such an isoform of MK2 is specifically addressed by the invention which plays a central role in the p38MAPK/MK2-dependent signaling pathway and, therefore, in the development of leukemia.

In a yet further embodiment of the invention the MK2 inhibitor is CMPD1.

CMPD1 (CAS. no.: 41179-33-3; MW: 349.4, C₂₂H₂₀FNO₂) is a selective and non-ATP-competitive p38 MAPK-mediated MK2 phosphorylation inhibitor with apparent Kᵢ (Kᵢ^{app}) of 330 nM.

The inventors have surprisingly realized in an experimental screening set up that CMPD1 induces a gene expression profile which is similar to the one that can be observed when knocking out the BAALC gene, thereby reversing the driving force of the development of AML. CMPD1 is, therefore, according to the findings of the inventors, one of the most effective MK2 inhibitors in the prophylaxis and treatment of leukemia, in particular of AML.

Another subject-matter of the invention is a pharmaceutical composition for the prophylaxis and/or treatment of leukemia and/or the development of leukemia, comprising the MK2 inhibitor according to the invention and a pharmaceutically acceptable carrier.

A "pharmaceutical composition" is a composition suitable for an administration to an animal and/or human being in a medical setting. Preferably, a pharmaceutical composition is sterile and produced according to GMP guidelines.

In an embodiment of the invention the MK2 inhibitor can be used as the only active agent of the pharmaceutical composition, i.e. in form of a monotherapeutics, without any additional further active agent.

The pharmaceutical composition comprises the MK2 inhibitors either in the free form or in the form of a pharmaceutically acceptable salt.

Pharmaceutically acceptable carriers are well known in the art and include, for example, aqueous solutions such as water or physiologically buffered saline or other solvents or vehicles such as glycols, glycerol, oils such as olive oil or injectable organic esters. In preferred embodiments, when such pharmaceutical compositions are for human administration, e.g., for parenteral administration, the aqueous solution is pyrogen-free, or substantially pyrogen-free. The excipients can be chosen, for example, to effect delayed release of an agent or to selectively target one or more cells, tissues or organs. The pharmaceutical composition can be in dosage unit form such as tablet, capsule (including sprinkle capsule and gelatin capsule), granule, powder, syrup, suppository, injection or the like. The composition can also be present in a transdermal delivery system, e.g., a skin patch. The composition can also be present in a solution suitable for topical administration, such as an eye drop. Alternatively, the pharmaceutical composition can be present in form of an aerosol administrable by inhalation.

The pharmaceutical composition as well as the methods to be explained below of the present invention may be utilized to treat a living being in need thereof. In certain embodiments, the living being is a mammal such as a human, or a non-human mammal.

In an embodiment of the pharmaceutical composition of the invention the MK2 inhibitor is present per dosage unit at a concentration which, upon administration into a living being, results in a concentration in said living being which inhibits the leukemogenesis and/or eliminates leukemia cells in said living being.

This measure has the advantage that the MK2 inhibitor is provided in said pharmaceutical composition in an adequate amount, thereby reducing potential side effects to a minimum.

The features, embodiments and advantages of the inhibitor according to the invention apply to the pharmaceutical composition in a corresponding manner, even if not specifically indicated.

The invention further relates to a method for the prophylaxis and/or treatment of leukemia and/or the development of leukemia in a living being, preferably a human being, comprising a step of inhibiting the leukemogenesis in said living being, wherein said inhibition of the leukemogenesis and/or elimination of leukemia cells is realized via the administration of an MK2 inhibitor to said living being.

The invention also relates to a method for the prophylaxis and/or treatment of leukemia and/or the development of leukemia in a living being, comprising a step of inhibiting the leukemogenesis in said living being, wherein said inhibition of the leukemogenesis is realized via the inhibition of BAALC in said living being, preferably knocking out of the gene encoding BAALC (BAALC KO) in said living being.

According to the invention "inhibition of BAALC" refers to any phenomenon which specifically and selectively reduces or abolishes the activity and/or functionality of BAALC in said living being, e.g. human being. This inhibition can be achieved, e.g. via the administration into the living being of a specific BAALC inhibitor, an siRNA inhibiting the translation of the coding molecule of BAALC, or, preferably, via knocking out the gene encoding BAALC (BAALC KO) etc.

The features, embodiments and advantages of the inhibitor according to the invention apply to the methods according to the invention in a corresponding manner, even if not specifically indicated.

In an embodiment of the method according to the invention the BAALC KO is realized via the use of proteases, preferably via CRISPR/Cas9 gene-editing.

This method has the advantage that the BAALC gene is knocked-out in a targeted manner, thereby limiting or even avoiding off-target effects.

The invention further relates to a 'single guide RNA' sgRNA molecule targeting the gene encoding BAALC in a living being, preferably a human being, for use in the prophylaxis and/or treatment of leukemia and/or the development of leukemia.

By this measure a tool is provided which can be used in the method according to the invention for knocking out of the gene encoding BAALC via CRISPR/Cas9 gene-editing.

According to the invention a "single guide RNA" (sgRNA) or guide RNA is a component of the CRISPR complex which serves to guide the CRISPR endonuclease to its target. The sgRNA is a non-coding short RNA sequence which binds to the complementary target DNA sequence of the BAALC gene. The sgRNA first binds to the CRISPR endonuclease enzyme and the sgRNA sequence guides the complex via pairing to the location on the DNA specifically encoding the BAALC gene. There the modified CRISPR endonuclease performs its nickase activity by cutting the target single DNA strand resulting in a knock-out of the BAALC gene.

Another subject-matter of the invention is a nucleic acid molecule, preferably the sgRNA molecule according to the invention, comprising any of the nucleotide sequences of SEQ ID NOS:1, 2, 39-78.

As the inventors were able to realize said nucleic acid molecule precisely cuts the BAALC encoding sequence on the DNA, thereby ensuring a knocking out of the BAALC gene resulting in the targeted inhibition of the leukemogenesis.

The features, characteristics, and embodiments of the inhibitor according to the invention apply to this sgRNA or nucleic acid molecule according to the invention in a corresponding manner, even if not specifically indicated.

In case of discrepancies between the nucleotide sequences disclosed in the application body and those disclosed in the sequence listing the nucleotide sequences in the application body have priority.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

The invention is now described and explained in further detail by referring to the following non-limiting examples and figures.

### BRIEF DESCRIPTION OF THE FIGURES

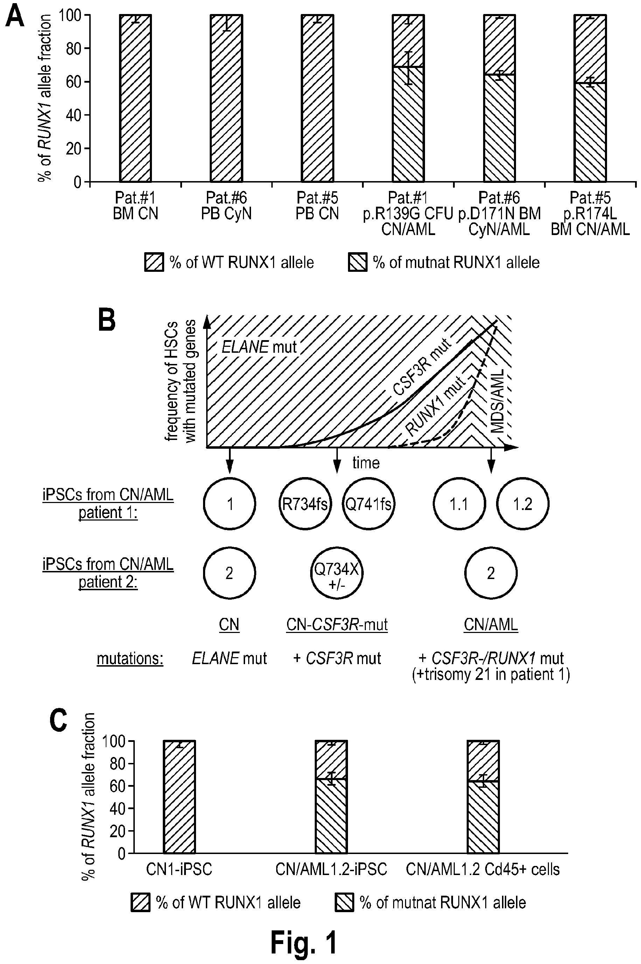

- Fig. 1:: Allele frequencies of missense RUNX1 mutations in CN/AML with trisomy 21. (A) Analysis of the allele frequency (AF) of wild type (WT) and corresponding mutant RUNX1 in CN patients prior- and after onset of AML (CN/AML). Each bar represents percentage of WT (green) and mutant (red) RUNX1 AF quantified by dPCR. In the CN phase, genotyping of BM or PB samples revealed an absence of mutant RUNX1 allele; whereas, in overt AML, 59-68% of leukemia cells acquired RUNX1 mutations, indicating the gain of a second mutant RUNX1 allele. Data are represented as mean ± SD. (B) A stepwise in vitro model of leukemogenesis in congenital neutropenia through the generation of patient-specific iPSCs and the insertion of additional mutations using CRISPR/Cas9 gene-editing. Top panel represents the time scale of leukemic transformation in CN/AML patients, the bottom panel represents the generation of single iPSC clones at specific time points during disease progression. (C) dPCR analysis of the allele frequency of WT and mutant (p.R139G) RUNX1 in CD45+ cells derived from CN1, CN/AML1.1 and CN/AML1.2 iPSCs.

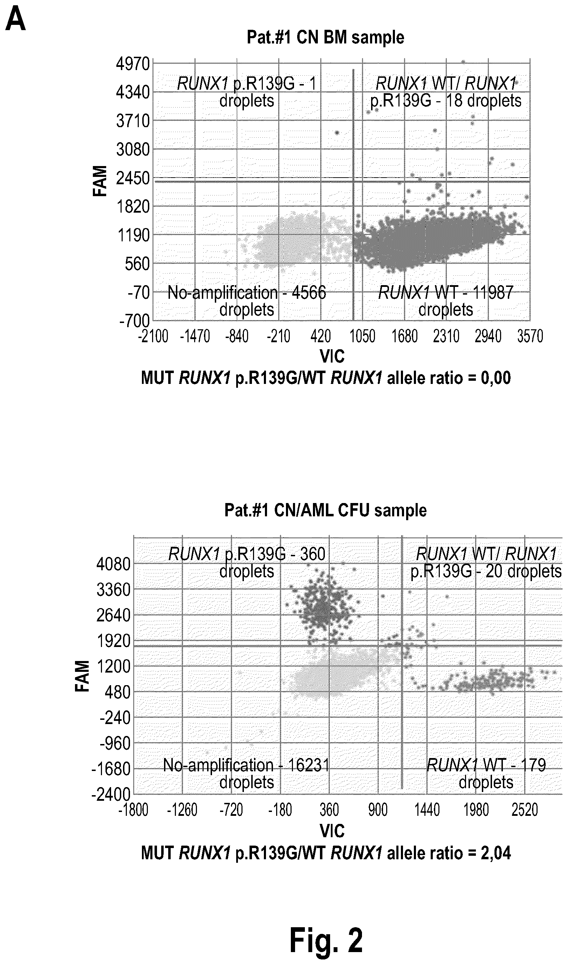

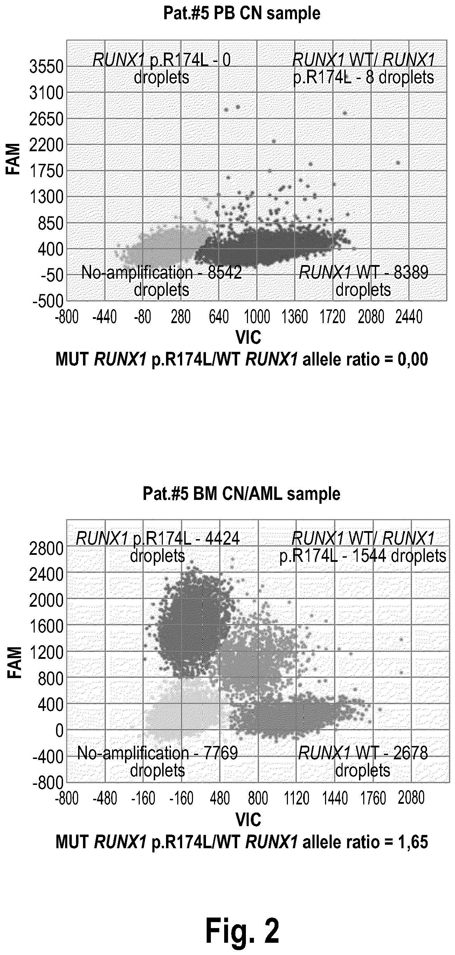

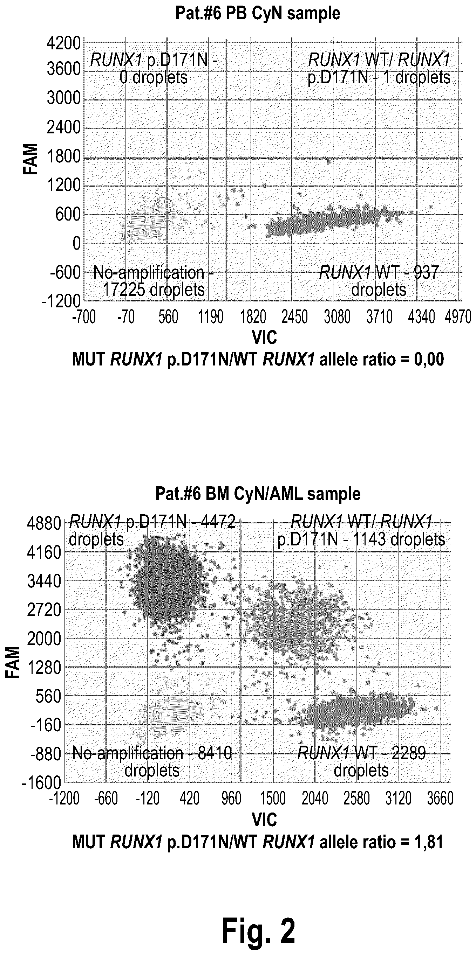

- Fig. 2:: Representative images of digital PCR data plots for quantification of RUNX1 allele ratio in CN and CN/AML samples, related to Figure 1. (A) The x-axis shows the signal from FAM reporter dye, and the y-axis shows the - the VIC reporter dye. Blue dots represent FAM-labeled mutant RUNX1 PCR product, red dots represent VIC-labeled WT RUNX1 PCR product, green dots represent droplets containing both mutant and WT DNA, and yellow dots are droplets with no DNA incorporated. Ratio of mutant and WT RUNX1 allele was calculated based on number of FAM and VIC positive signals on the chips. For example, for Pat.#1 DNA isolated from BM (CN-phase) and DNA extracted from single-cell derived CFU clones (CN/AML phase) were amplified using TaqMan assay probe set against WT RUNX1 and RUNX1 p.R139G allele. The presence of RUNX1 p.R139G mutation was detected in 360 droplets, representing an allelic fraction of 66.8 %. Correspondingly, the ratio of mutant to WT allele is 2.04.

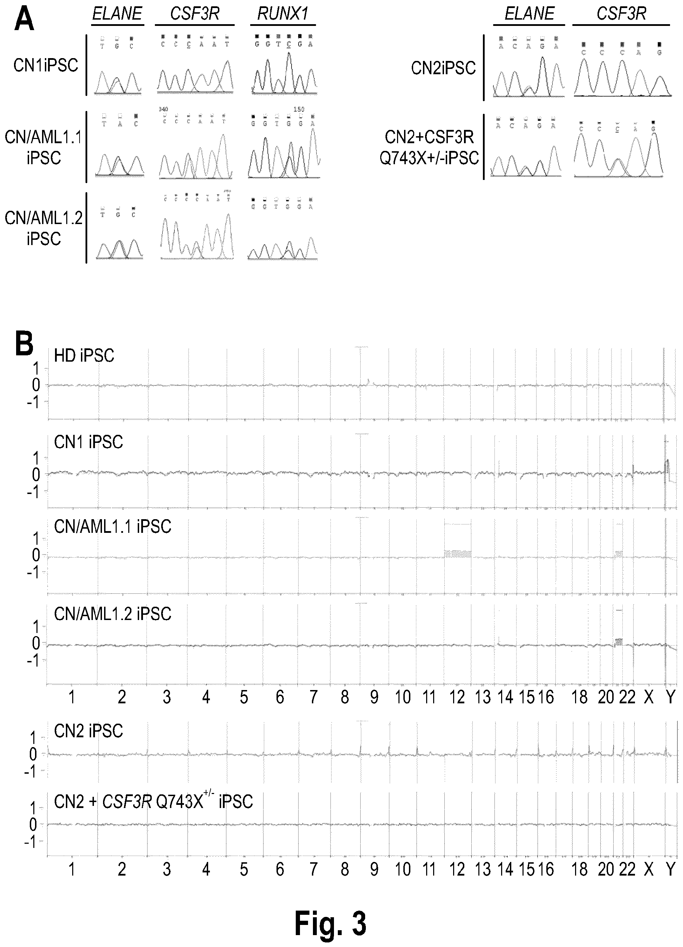

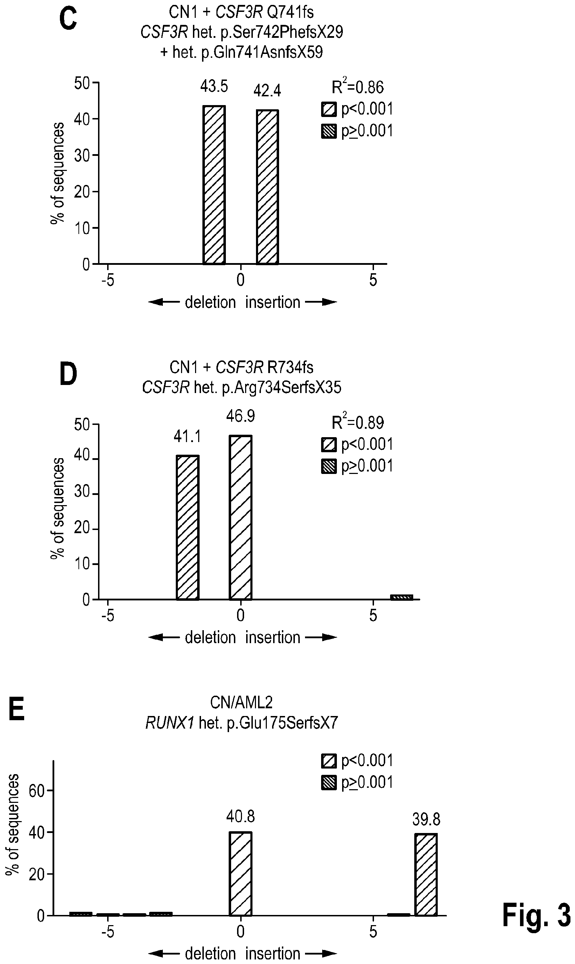

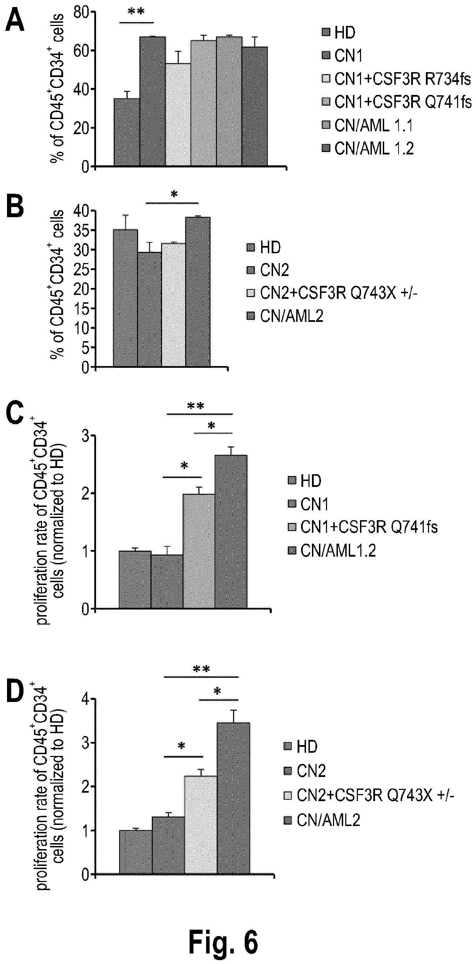

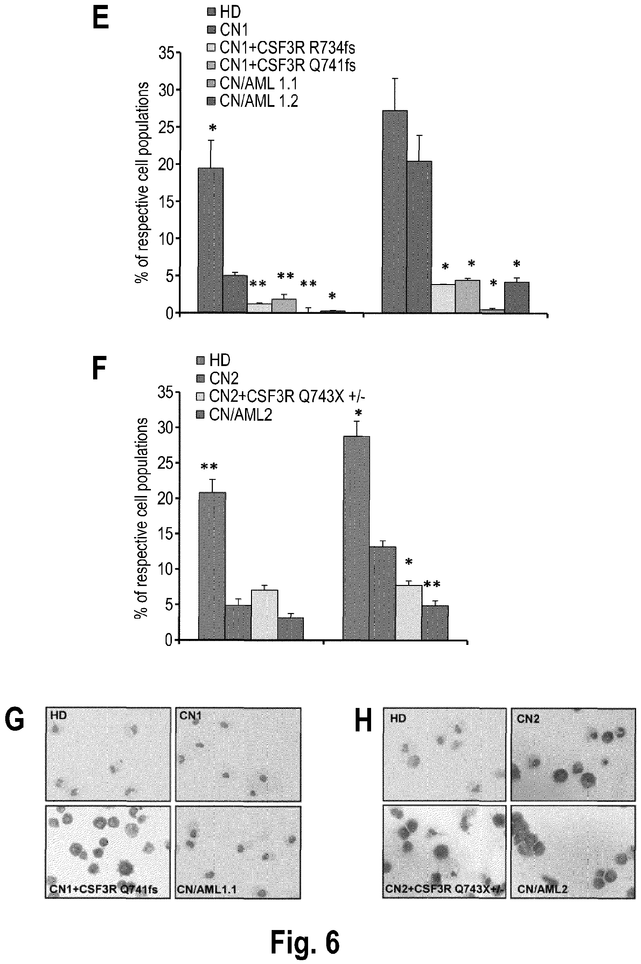

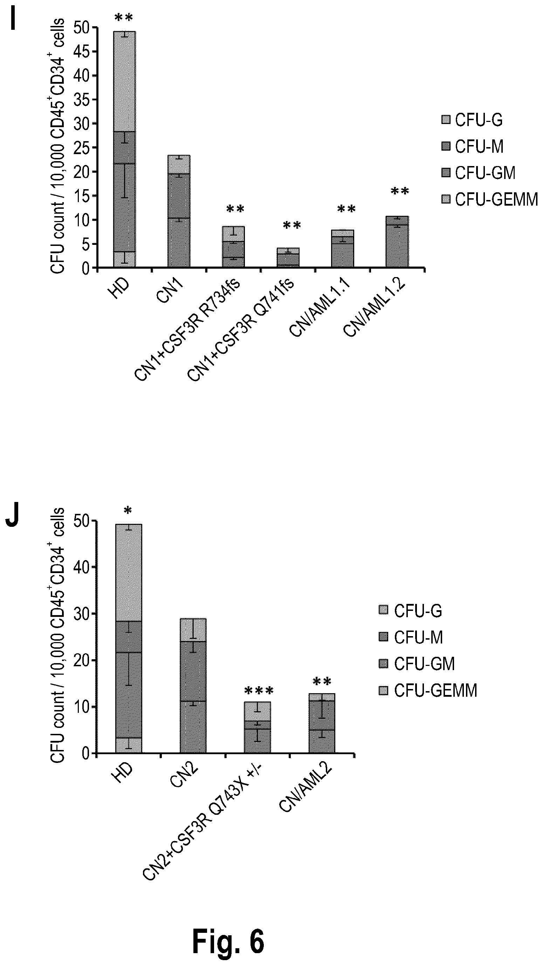

- Fig. 3:: Genetic analysis of iPSCs clones derived from CN/AML patients, related to Figure 1 and 6. (A) Representative Sanger Sequencing diagrams of DNA isolated from indicated iPSC clones confirming ELANE p.C151R, CSF3R p.Q741X and RUNX1 p.R139G heterozygous mutations for CN/AML patient 1, and ELANE p.G214R and CSF3R p.Q743X heterozygous mutations for CN/AML patient 2. (B) Representative images of array CGH of indicated iPSC clones. Whole genome view is shown. (C-E) Assessment of CRISPR/Cas9 gene-edited iPSC clones. DNA isolated from single-cell derived iPSC clones was amplified by PCR and analyzed using TIDE (Tracking of Indels by DE-composition). Graphs show the estimated composition of the inserted/deleted (in/del) nucleotide bases in iPSC lines calculated by TIDE. Additionally, genetic changes introduced by CRISPR/Cas9 have been annotated at protein level in order to predict the consequence of in/del introduced by genome editing in the amino acid sequences of modified genes.

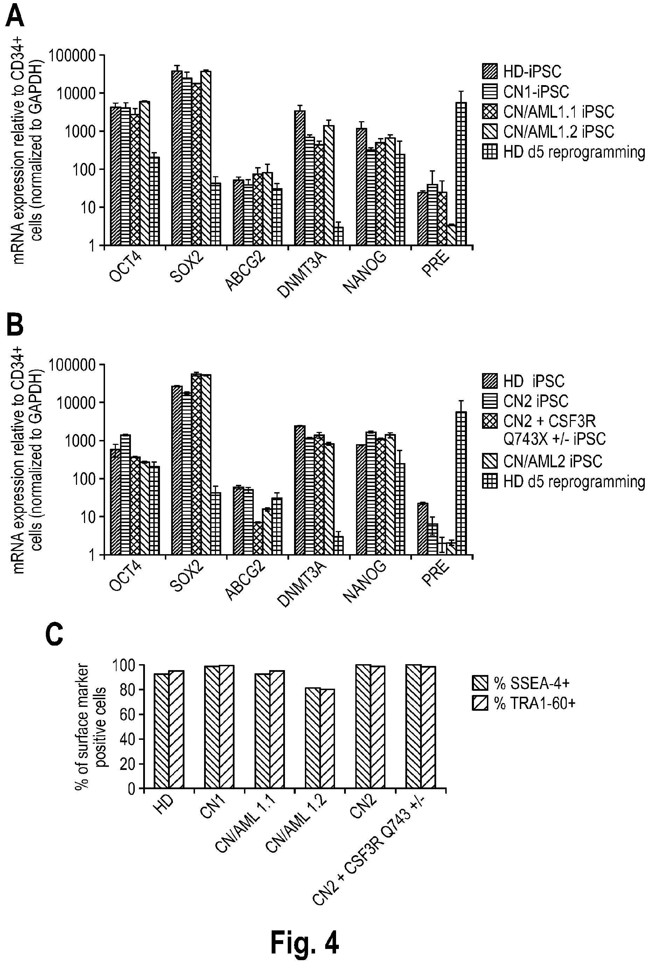

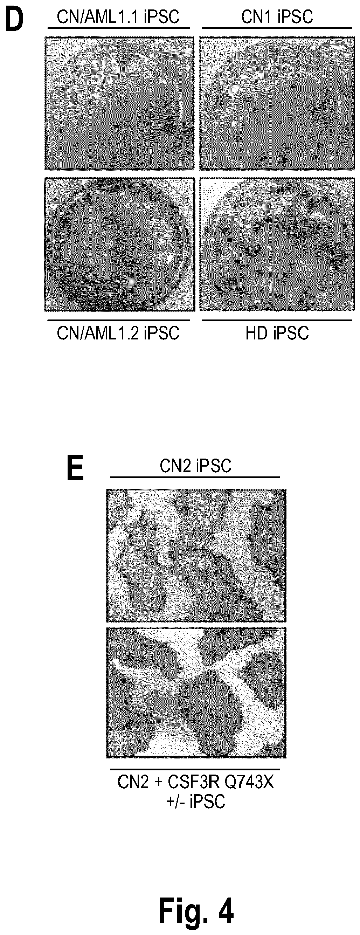

- Fig. 4:: Characterization of HD, CN and CN/AML iPSC clones, related to Figure 6. (A, B) qRT-PCR analysis of mRNA expression of pluripotency-specific genes using mRNA isolated from indicated iPSC clones of CN/AML patient 1 (A) and patient 2 (B). Gene expression levels were normalized to GAPDH and shown relative to HD CD34+ cells. PRE indicates exogenous mRNA expression of the lentiviral vector. Data are represented as mean ± SD; two independent experiments. (C) Percentage of pluripotency-specific surface markers (Tra1-60 and SSEA4) in indicated iPSC clones of CN/AML patient 1 and 2. (D, E) Representative images of the Alkaline Phosphatase activity (purple) analysis in iPSC colonies of CN/AML patient 1 (D) and patient 2 (E).

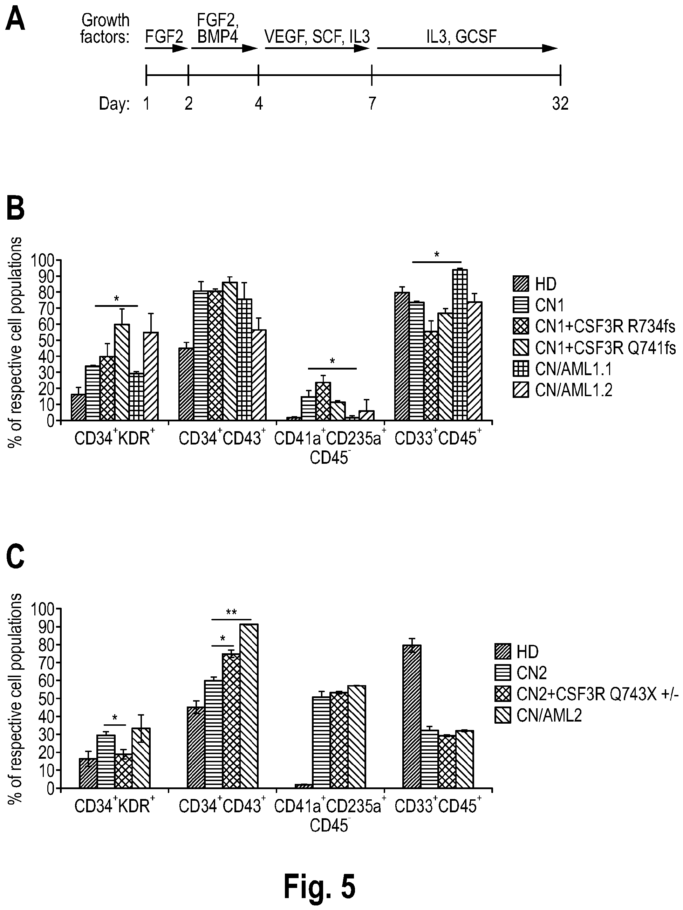

- Fig. 5:: Analysis of hematopoietic differentiation of CN and CN/AML iPSC-derived HSPCs by flow cytometry, related to Figure 6. (A) Schematic overview of the EB-based hematopoietic/neutrophilic differentiation of iPSCs. (B, C) Percentage of iPSC-derived immature hematopoietic cells (CD34⁺KDR⁺, CD34⁺CD43⁺, CD41a⁺CD235a⁺CD45⁺, CD33⁺CD45⁺) in CN/AML patient 1 (B) and patient 2 (C). iPSCs were cultured under EB differentiation conditions for 14 days. Data are represented as mean ± SD; two independent experiments; including group comparisons. Significant difference between groups to CN is indicated, *P < 0.05, **P < 0.01, ***P < 0.001.
- Fig. 6:: Hematopoietic differentiation of CN/AML patients derived iPSCs. (A, B) Percentage of CD34⁺ cells derived from CN/AML1 (A) and CN/AML2 (B) iP-SCs on day 14 of culture. Data are represented as mean ± SD; two independent experiments. Group comparisons were done between CN and all other iPSC clones. Significant difference between groups to CN is indicated, *P < 0.05, **P < 0.01. (C, D) Proliferation rate of CD34⁺ cells derived from CN/AML1 (C) and CN/AML2 (D) iPSCs on day 14 of culture. HSPCs were expanded on SL/SL feeder for 7 days. Data were normalized to healthy donor (HD) control and are represented as mean ± SD; two independent experiments. Significant difference between groups to CN is indicated, *P < 0.05, **P < 0.01. (E, F) Percentage of iPSC-derived granulocytes (CD15⁺CD16⁺CD45⁺ and CD15⁺CD11b⁺CD45⁺) in CN/AML1 (E) and CN/AML2 (F). iPSCs were cultured under EB differentiation conditions for 32 days. Data are represented as mean ± SD from two independent experiments. Group comparisons were done between CN and all other iPSC clones. Significant difference between groups to CN is indicated, *P < 0.05, **P < 0.01. (G, H) Representative images of iPSC-derived hematopoietic cells at day 32 of differentiation stained with Wright Giemsa are shown for CN/AML1 (G) and CN/AML2 (H). 40x magnification is shown. (I, J) CFU assay of iPSC-derived CD34⁺ cells isolated from EBs culture at day 14. Total CFU counts are shown for CN/AML1 (I) and CN/AML2 (J). Data are represented as mean ± SD from two independent experiments in triplicate. Group comparisons were done between CN and all other iPSC clones. Significant difference between groups to CN is indicated, *P < 0.05, **P < 0.01, ***P < 0.001.

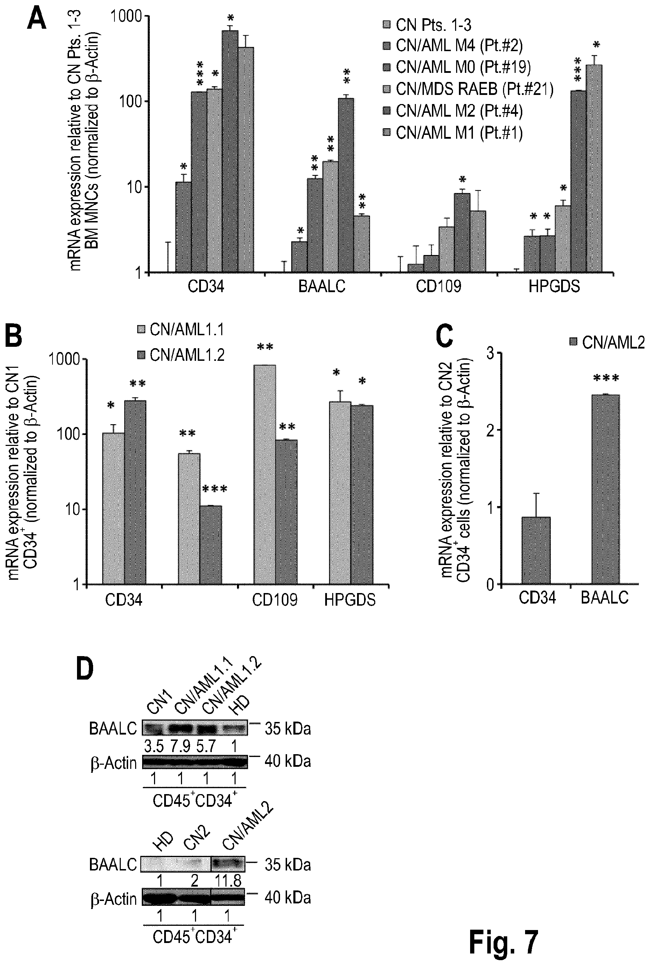

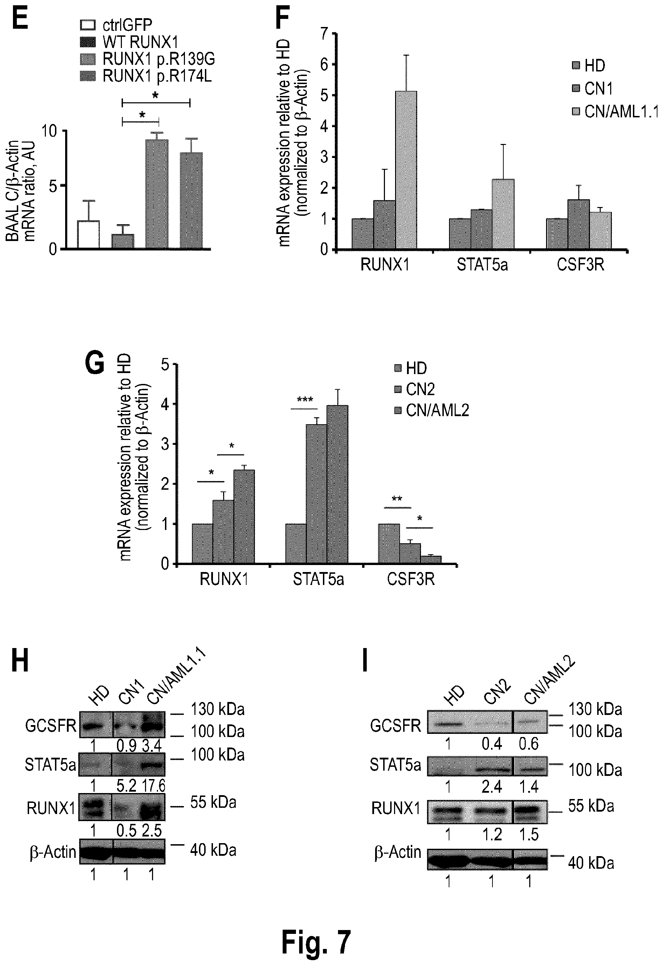

- Fig. 7:: Up-regulation of AML-associated genes in primary CN/AML blasts and CN/AML iPSC-derived CD34⁺ cells. (A) mRNA expression of indicated genes in BMMNCs from three CN and five CN/AML patients, measured by qPT-PCR. Gene expression levels were normalized to β-actin and shown relative to CN. Data are represented as mean ± SD from two independent experiments. *P < 0.05, **P < 0.01, ***P < 0.001 compared to CN. (B, C) mRNA expression of indicated genes in CD34⁺ cells derived from CN1 and CN/AML1 (B) as well as CN2 and CN/AML2 (C) iPSCs, measured by qPT-PCR. Gene expression levels were normalized to β-actin and shown relative to CN. Data are represented as mean ± SD; two independent experiments. *P < 0.05, **P < 0.01, ***P < 0.001 compared to CN. (D) Western blotting of BAALC protein expression in CD34+ cells derived from HD, CN, CN/AML iPSCs. Numbers indicate protein expression levels were normalized to β-actin and shown relative to HD. The results are representative of two independent experiments. (E) mRNA expression of BAALC in CD34⁺ cells from healthy individuals (n = 2) transduced with control GFP, WT RUNX1 or mutant RUNX1 (p.R139G and p.R174L) lentiviral constructs, measured by qPT-PCR. Gene expression levels were normalized to β-actin. Data are represented as mean ± SD from two independent experiments. *P < 0.05. (F, G) mRNA expression of indicated genes in CD34+ cells derived from (F) HD, CN1 and CN/AML1 and (G) HD, CN2 and CN/AML2 iP-SCs. Gene expression levels were normalized to β-actin and shown relative to HD. Data are represented as mean ± SD from two independent experiments. *P < 0.05, **P < 0.01, ***P < 0.001. (H, I) Western blotting of indicated proteins in CD34+ cells derived from (H) HD, CN1 and CN/AML1 and (I) HD, CN2 and CN/AML2 iPSCs. Numbers indicate protein expression levels relative to HD and normalized to β-actin. The results are representative of two independent experiments.

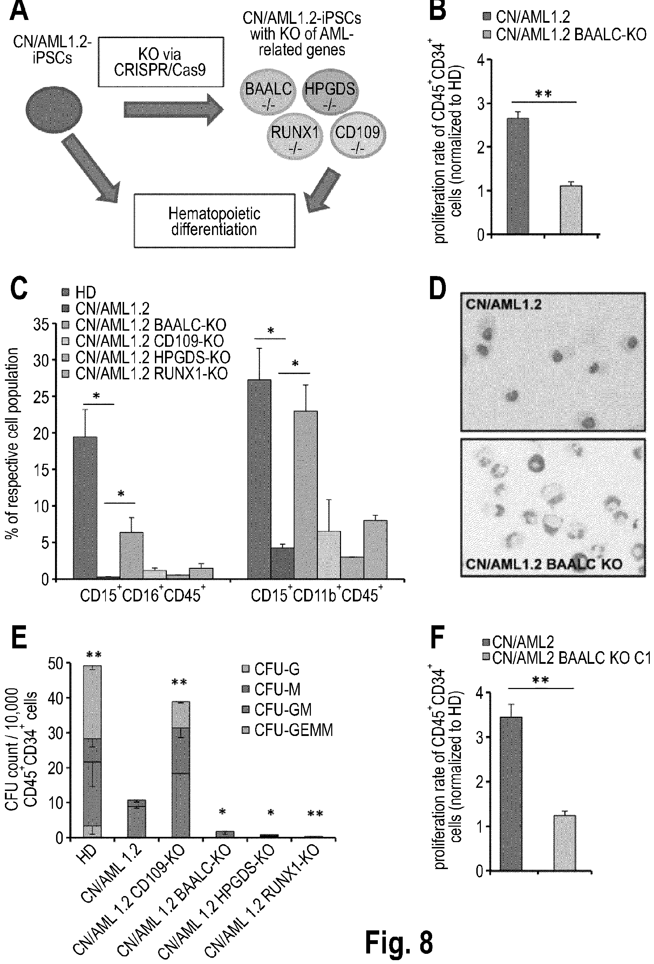

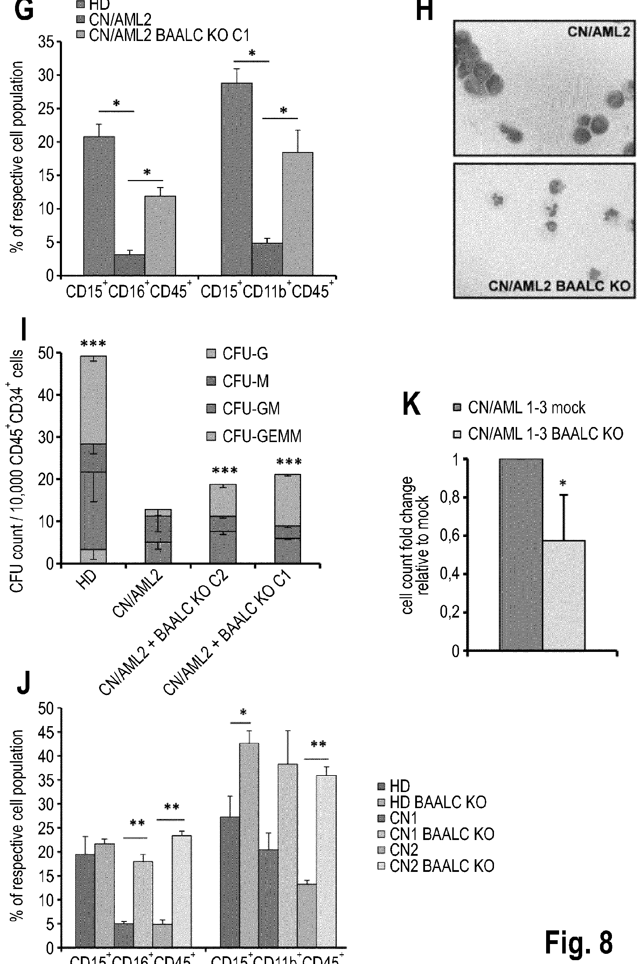

- Fig. 8:: BAALC upregulation is essential for leukemogenic transformation in CN/AML. (A) Schematic overview of the experimental design. CRISPR/Cas9-mediated gene-editing of indicated genes was performed in CN/AML iPSCs followed by EB-based differentiation. Hematopoietic differentiation was compared to unedited CN/AML iPSCs and HD iPSCs. (B) Proliferation rate of CD34⁺ cells derived from CN/AML1 and CN/AML1 BAALC KO iPSCs. CD34⁺ cells were expanded on SL/SL feeder for 7 days. Data were normalized to HD and are represented as mean ± SD from two independent experiments. **P < 0.01. (C) Percentage of iPSCs derived granulocytes (CD15⁺CD16⁺ CD45⁺ and CD15⁺CD11b⁺CD45⁺) on day 32 of culture. Data are represented as mean ± SD from two independent experiments. *P < 0.05. (D) Representative images of iPSC-derived hematopoietic cells at day 32 of differentiation stained with Wright Giemsa at 40x magnification are shown. (E) CFU assay of iPSCs-derived CD34⁺ cells isolated at day 14 of culture. Total CFU counts are shown. Data are represented as mean ± SD from two independent experiments in trip-licate. Group comparisons were done between CN/AML1 and all other iPSC clones. Significant difference between groups to CN/AML1 is indicated, *P < 0.05, **P < 0.01. (F) Proliferation rate of CD34⁺ cells derived from CN/AML2 and CN/AML2 BAALC KO iPSCs. CD34⁺ cells were expanded on SL/SL feeder for 7 days. Data were normalized to HD and are represented as mean ± SD from two independent experiments. **P < 0.01. (G) Percentage of iPSC-derived granulocytes (CD15⁺CD16⁺CD45⁺ and CD15⁺CD11b⁺CD45⁺) on day 32 of culture. Data are represented as mean ± SD from two independent experiments. *P < 0.05. (H) Representative images of iPSC-derived hematopoietic cells at day 32 of differentiation stained with Wright Giemsa at 40x magnification are shown. (I) CFU assay of iPSCs-derived CD34⁺ cells isolated at day 14 of culture. Total CFU counts are shown. Data are represented as mean ± SD from two independent experiments in triplicate. Group comparisons were done between CN/AML2 and all other iPSC clones. Significant difference between groups to CN/AML2 is indicated, *P < 0.05, **P < 0.01, ***P < 0.001. (J) Percentage of iPSC-derived granulocytes (CD15⁺CD16⁺CD45⁺, CD15+CD11b+CD45+) on day 32 of culture. Data are represented as mean ± SD from two independent experiments. *P < 0.05, **P < 0.01. (K) Proliferation rate of primary CN/AML mock and CN/AML BAALC KO blasts from three CN/AML patients. Gene-edited primary CN/AML blasts were expanded on SL/SL feeder for 14 days. Data were normalized to mock cells and are represented as mean ± SD, *P < 0.05.

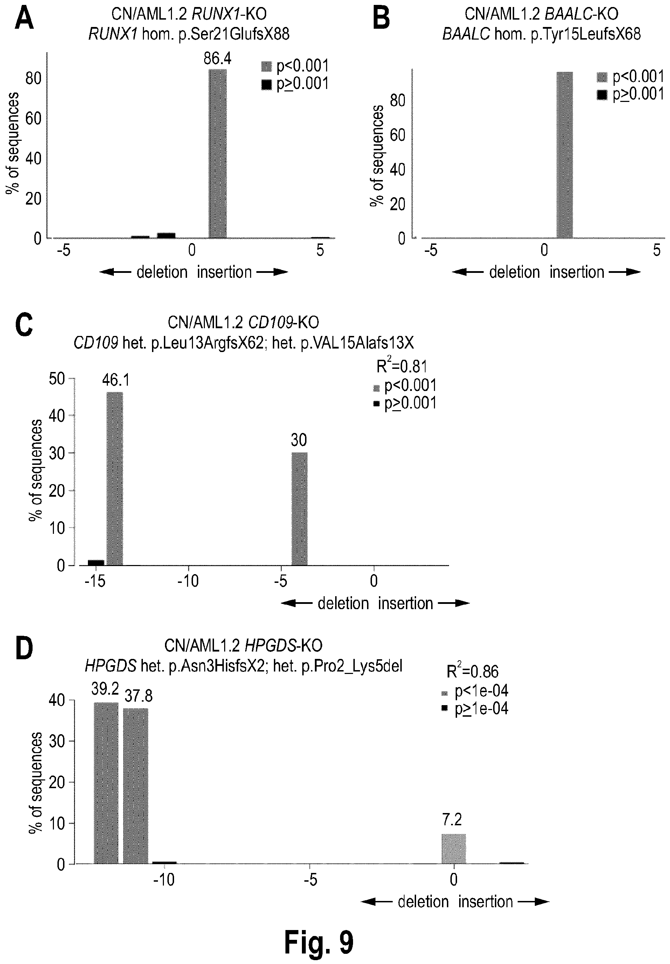

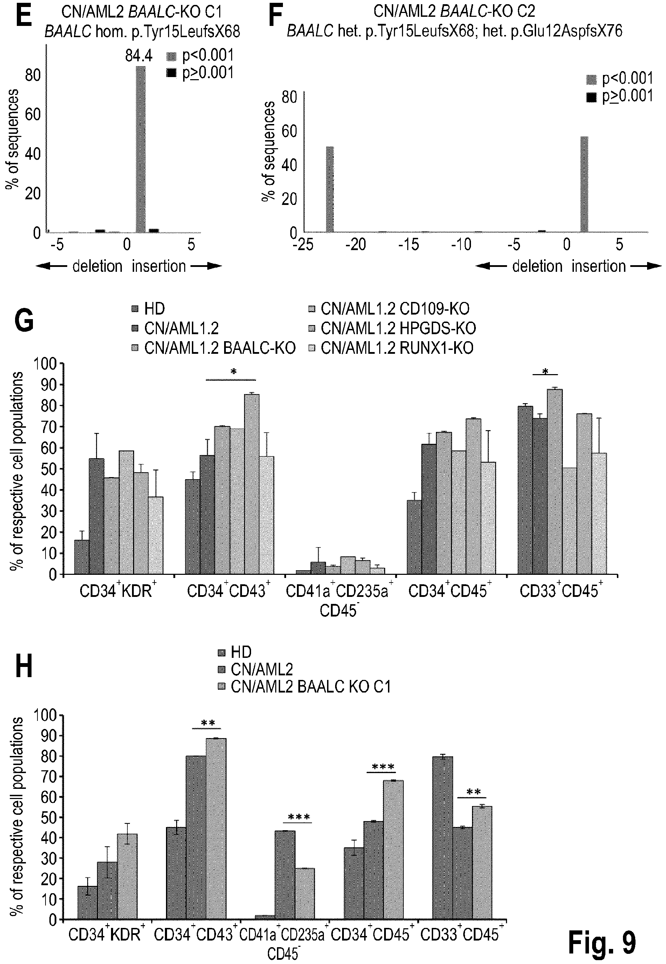

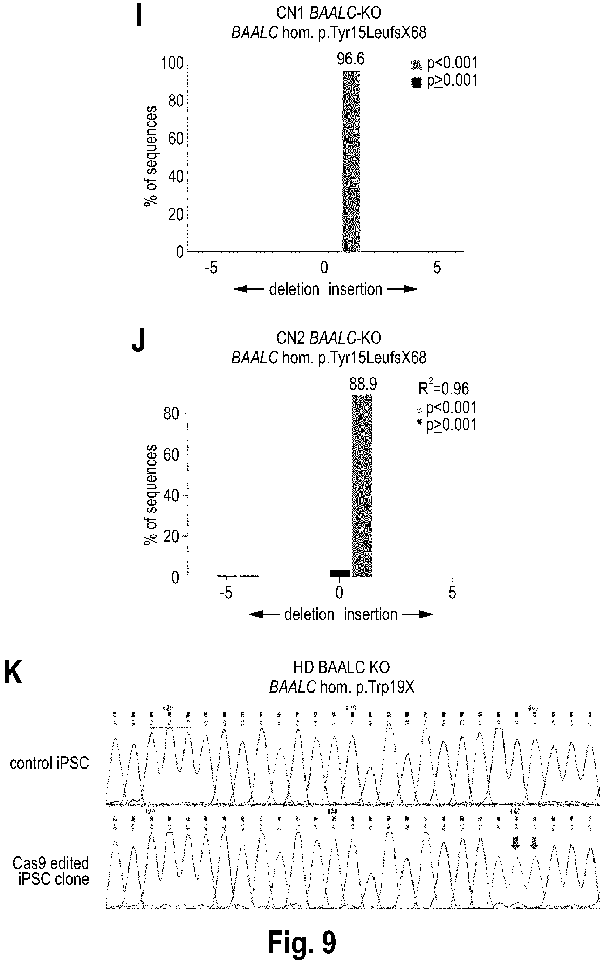

- Fig. 9:: Assessment of CRISPR/Cas9 gene-edited knockout iPSC lines and further analysis of hematopoietic differentiation in CN/AML iPSC knockout lines, related to Figure 8. (A-F, I-J) DNA isolated from single-cell derived iPSC clones was amplified by PCR and analyzed using TIDE (Tracking of Indels by Decomposition). Graphs show the estimated composition of inserted/deleted (in/del) nucleotide bases in iPSC lines calculated by TIDE. Additionally, genetic changes introduced by CRISPR/Cas9 have been annotated at protein level in order to predict the consequence of in/dels introduced by genome editing in the amino acid sequences of modified genes. (G, H) Percentage of iPSC-derived immature hematopoietic cells (CD34⁺KDR⁺, CD34⁺CD43⁺, CD41a⁺CD235a⁺CD45⁺, CD33⁺CD45⁺) in CN/AML iPSC KO clones of patient 1 (G) and patient 2 (H). iPSCs were cultured under EB differentiation conditions for 14 days. Data are represented as mean ± SD; two independent experiments; including group comparisons. Significant difference between groups to CN is indicated, *P < 0.05, **P < 0.01, ***P < 0.001. (K) Alignment of Sanger sequencing traces from parent control iPSC line (upper panel) and base edited HD BAALC KO (lower panel) confirmed the introduction of BAALC stop codon mutation in a single-cell derived iPSC clone. Positions of based edited nucleotides are depicted with black arrows. PAM sequence of guide RNA is marked above the alignment with a red line.

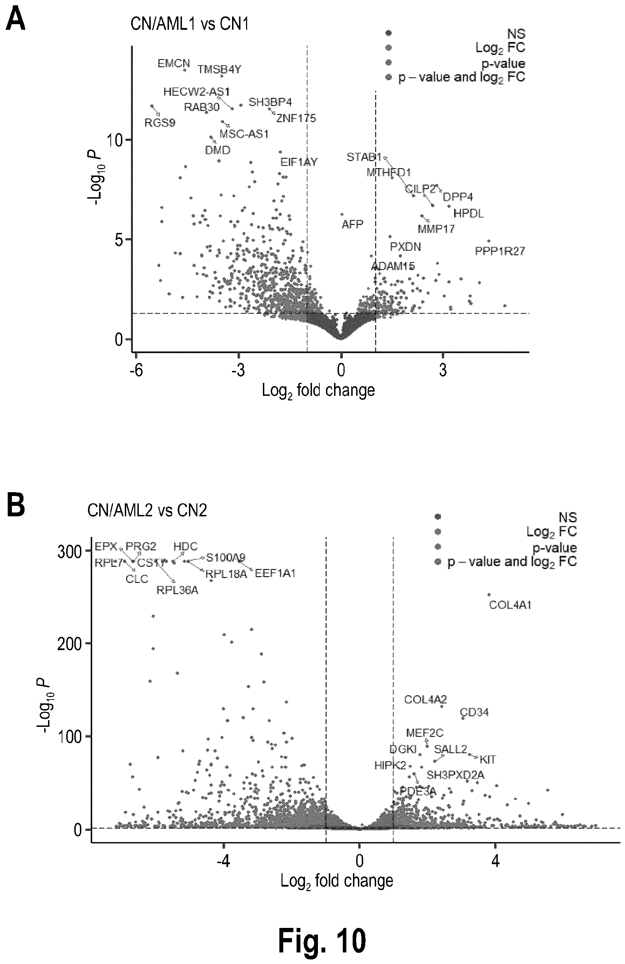

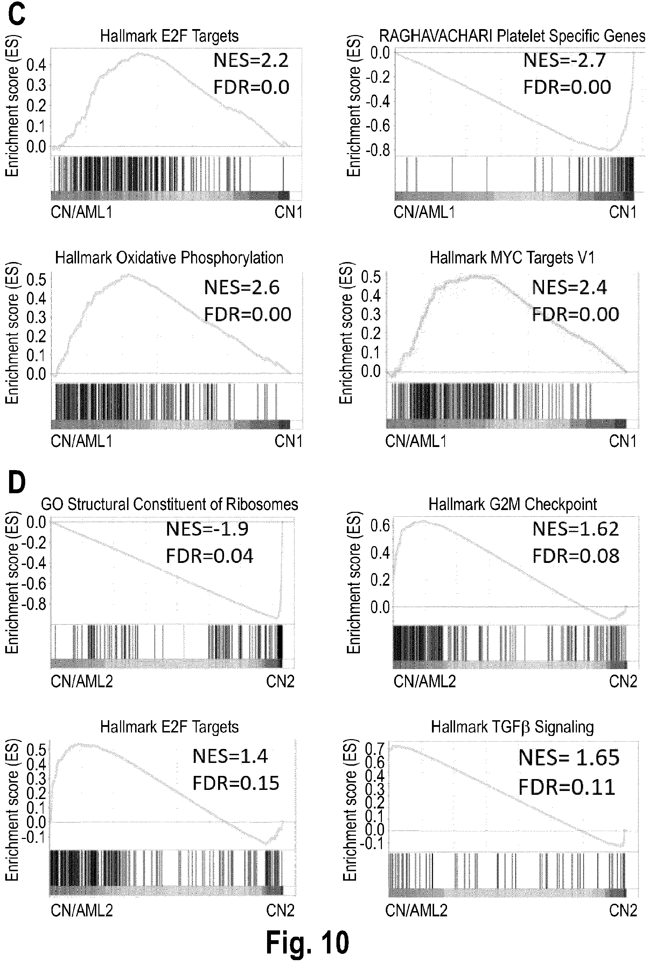

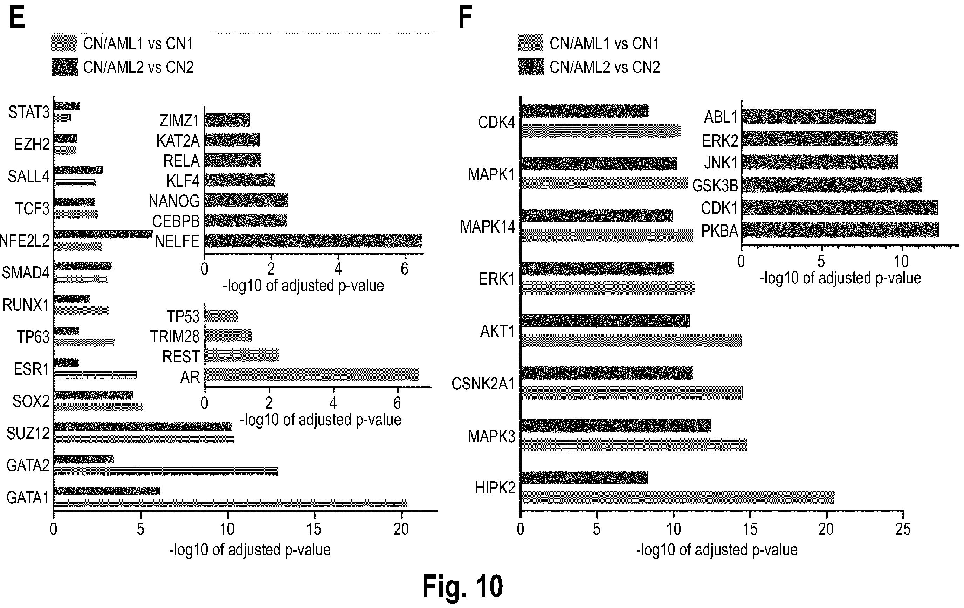

- Fig. 10:: Gene expression signature of CD34⁺ cells derived from CN and CN/AML iPSCs. (A, B) Volcano plot showing differentially expressed genes (DEGs, log₂FC > 1 or < -1, adjusted P-value < 0.05) in iPSC-derived CD34⁺ cells from (A) CN/AML1 vs CN1 and (B) CN/AML2 vs CN2 group comparisons. The x-axis shows the log2 fold change, and the y-axis shows the -log10 adjusted P-value. Colors represent the significance of the genes in terms of P-value and log₂ fold change. Gene names are indicated for the top 10 upregulated and top 10 downregulated genes. (C, D) GSEA 4.0.3 analysis of normalized read counts of iPSC-derived HSPCs from CN/AML1 vs CN1 (C) and CN/AML2 vs CN2 (D) groups. (E) Transcription factors predicted by TFEA of DEGs in CN/AML1 vs CN1 (red) and CN/AML2 vs CN2 (blue) groups. Common transcription factors are plotted on the same graph. Data are displayed as -log10 adjusted P-value. (F) Kinases predicted by KEA of DEGs in CN/AML1 vs CN1 (red) and CN/AML2 vs CN2 (blue). Common kinases with P-value < 10⁻⁸ are plotted on the same graph. Data are displayed as -log10 adjusted P-value.

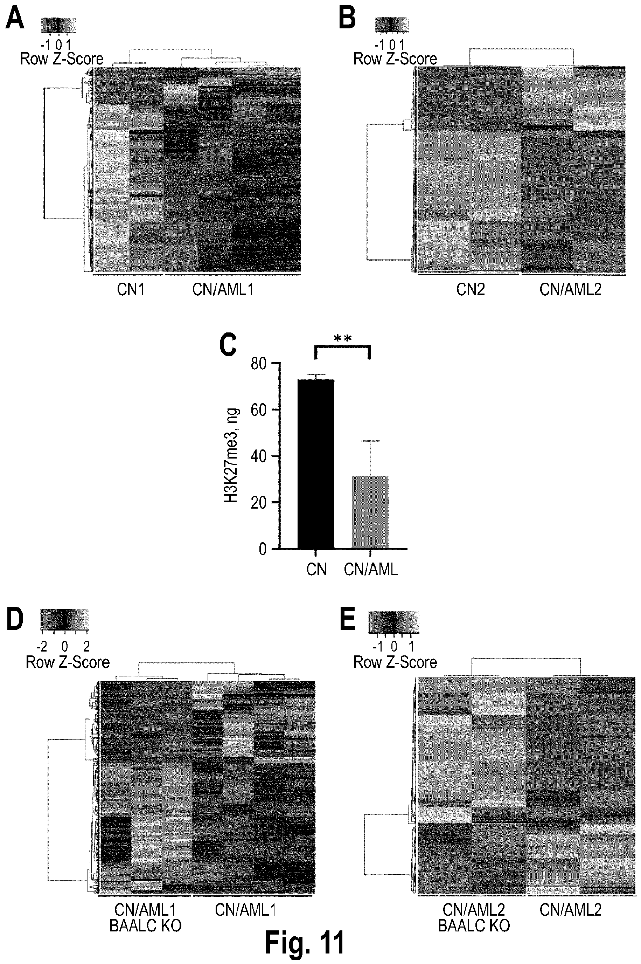

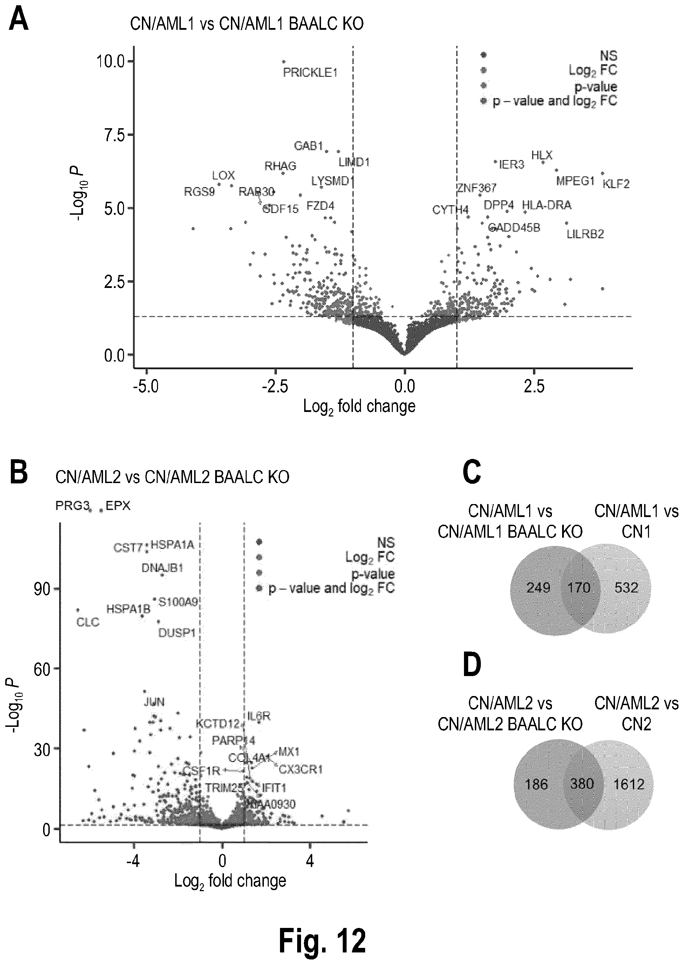

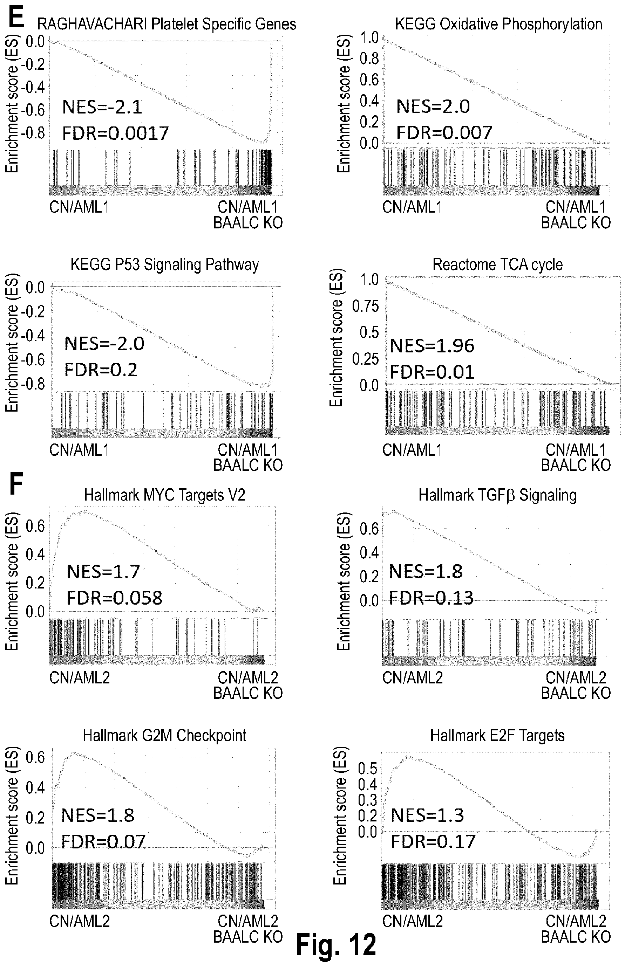

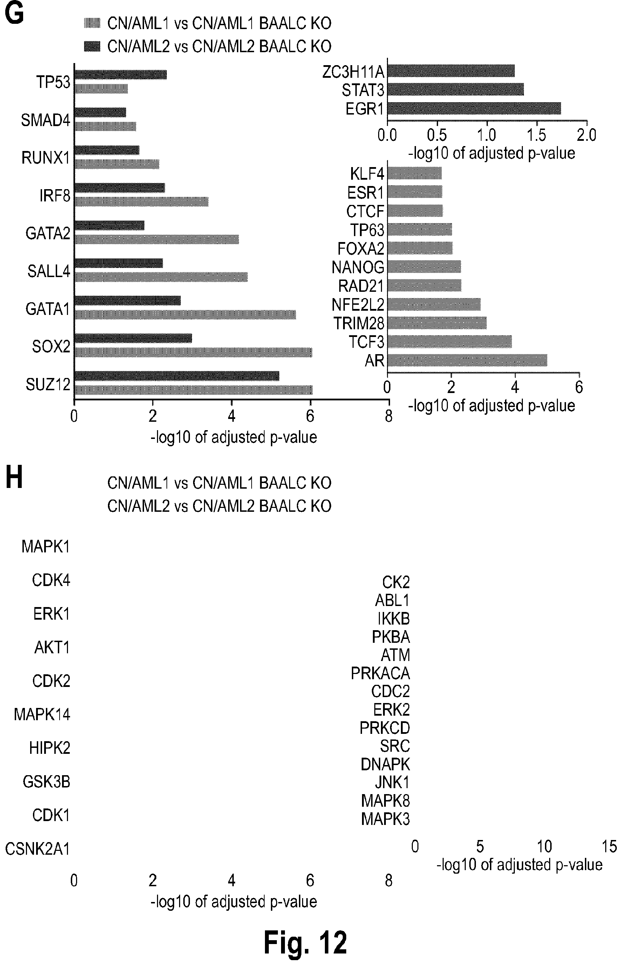

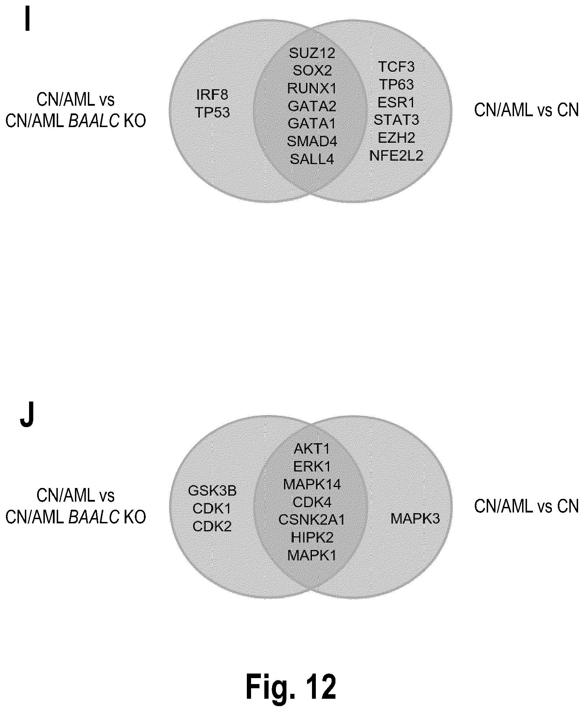

- Fig. 11:: Gene expression and histone H3 trimethyl Lys27 (H3K27me3) analysis of iPSC-derived HSPCs, related to Figure 10 and 12. (A) Supervised average linkage clustering of 702 differentially expressed genes (log₂FC > 1 or < -1, adj. P-value < 0.05) between iPSC-derived HSPCs from CN/AML1 and CN1 RNA-seq samples were obtained from 3 independent EB-based hematopoietic differentiation experiments performed with CN1, CN/AML1.1, or CN/AML 1.2 iPSC clones. Expression heatmap was created using variance stabilizing transformed read counts as an input. (B) Supervised average linkage clustering of 1992 differentially expressed genes (log₂FC > 1 or < -1, adj. P-value < 0.05) between iPSC-derived HSPCs from CN/AML2 and CN2. RNA-seq samples were obtained from 2 independent EB-based hematopoietic differentiation experiments performed with CN2 or CN/AML2 iPSC clones. Gene expression heatmap was created using as input variance stabilizing transformed read count data. (C) Total histone H3 trimethyl Lys27 (H3K27me3) in iPSC-derived HSPCs from CN and CN/AML clones of both patients. Data are represented as mean ± SD; duplicates/patient, **P < 0.001. (D) Supervised average linkage clustering of 419 differentially expressed genes (log₂FC > 1 or < -1, adjusted P-value < 0.05) between iPSC-derived HSPCs from CN/AML1 and CN1 BAALC KO. RNA-seq samples were obtained from 3 independent EB-based hematopoietic differentiation experiments performed with CN/AML1.2 and CN/AML1.2 BAALC KO iPSC clones. Gene expression heatmap was created using variance stabilizing transformed read counts as an input. (E) Supervised average linkage clustering of 566 differentially expressed genes (log₂FC > 1 or log₂FC < -1, adjusted P-value < 0.05) between iPSC-derived HSPCs from CN/AML2 and CN2 BAALC KO. RNA-seq samples were obtained from 2 independent EB-based hematopoietic differentiation experiments performed with CN/AML2 and CN/AML2 BAALC KO iPSC clones. Gene expression heatmap was created using variance stabilizing transformed read counts as an input.
- Fig. 12:: BAALC-dependent gene expression profile of CN/AML iPSC-derived CD34⁺ cells. (A, B) Volcano plot showing DEGs in iPSC derived CD34⁺ cells from (A) CN/AML1 vs CN/AML1 BAALC KO and (B) CN/AML2 vs CN/AML2 BAALC KO group comparisons. The x-axis shows the log₂ fold change, and the y- axis shows the -log10 adjusted P-value. Colors represent the significance of the genes in terms of P-value and log₂ fold change. Gene names are indicated for the top 10 upregulated and top 10 downregulated genes. (C, D) Venn diagram showing overlap of DEGs between (C) CN/AML1 vs CN/AML1 BAALC KO (pink) and CN/AML1 vs CN1 (blue) groups, as well as (D) CN/AML2 vs CN/AML2 BAALC KO (pink) and CN/AML2 vs CN2 (blue) groups. (E, F) GSEA 4.0.3 analysis of normalized read counts of RNA-seq from iPSC-derived HSPCs from (E) CN/AML1 vs CN/AML1 BAALC KO and (F) CN/AML2 vs CN/AML BAALC KO groups. (G) Transcription factors predicted by TFEA of DEGs in CN/AML1 vs CN/AML1 BAALC KO (red) and CN/AML2 vs CN/AML2 BAALC KO (blue) groups. Common transcription factors (P-value < 0.05) are plotted on the same graph. Data are displayed as -log10 of adjusted P-value. (H) Kinases predicted by KEA of DEGs in CN/AML1 vs CN/AML1 BAALC KO (red) and CN/AML2 vs CN/AML2 BAALC KO (blue) groups. Common kinases (P-value < 10-8) are plotted on the same graph. Data are displayed as -log10 of adjusted P-value. (I, J) Venn diagram showing overlap of transcription factors (I) and kinases (J) between CN/AML vs CN/AML BAALC KO (pink) and CN/AML vs CN (blue) groups of both CN/AML patients.

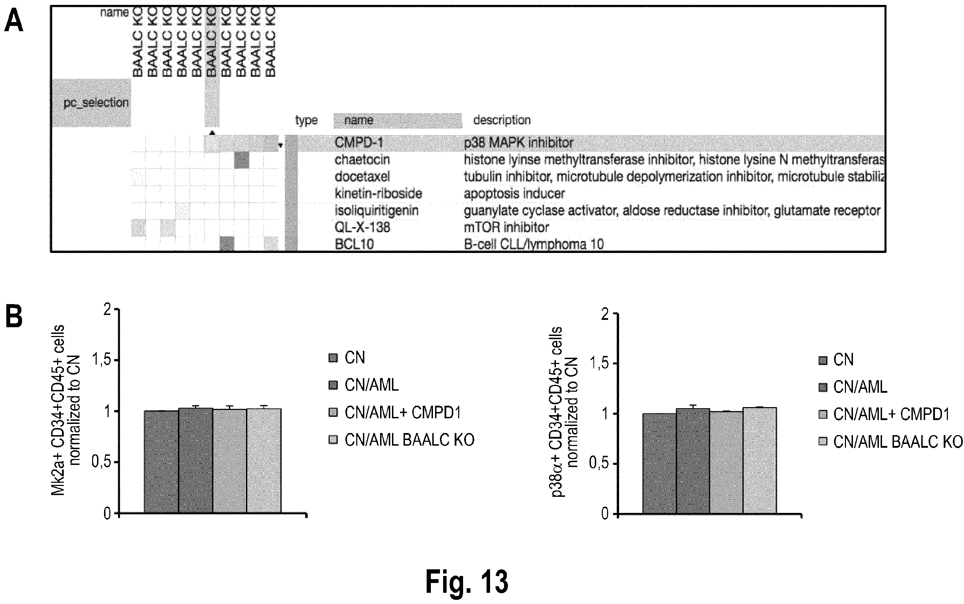

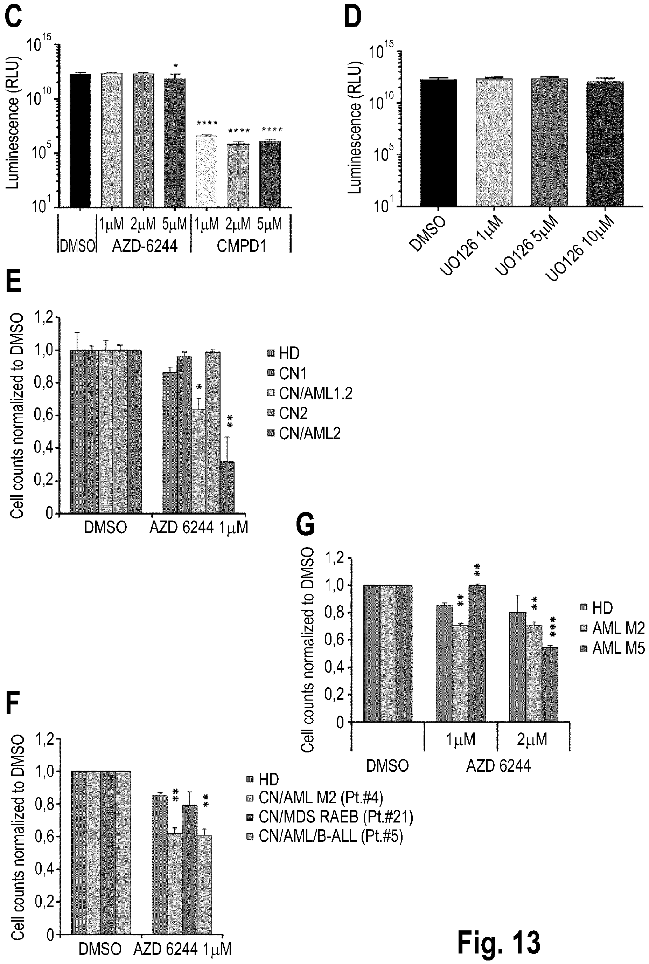

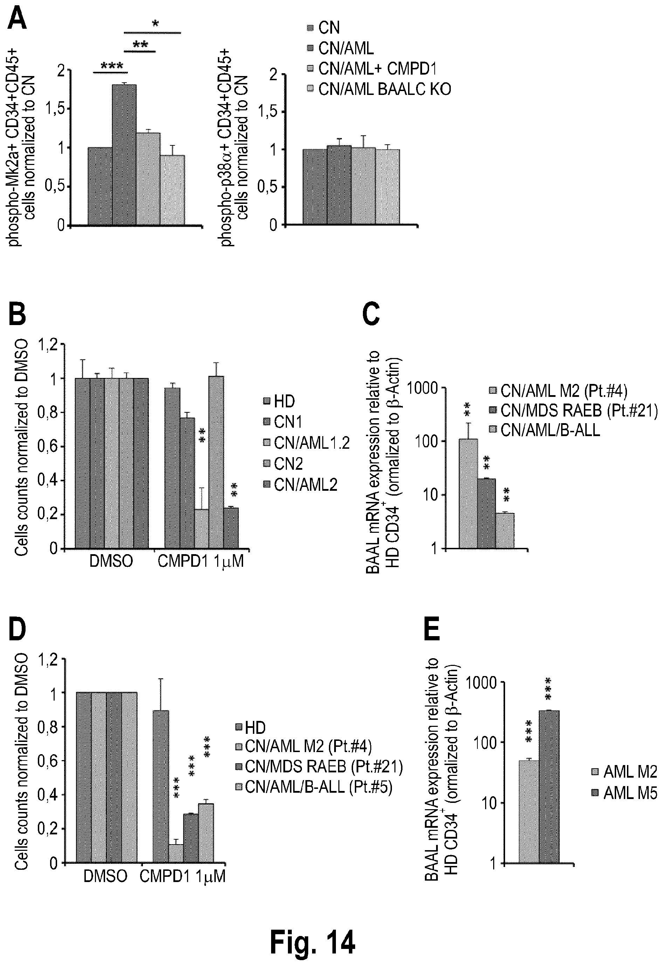

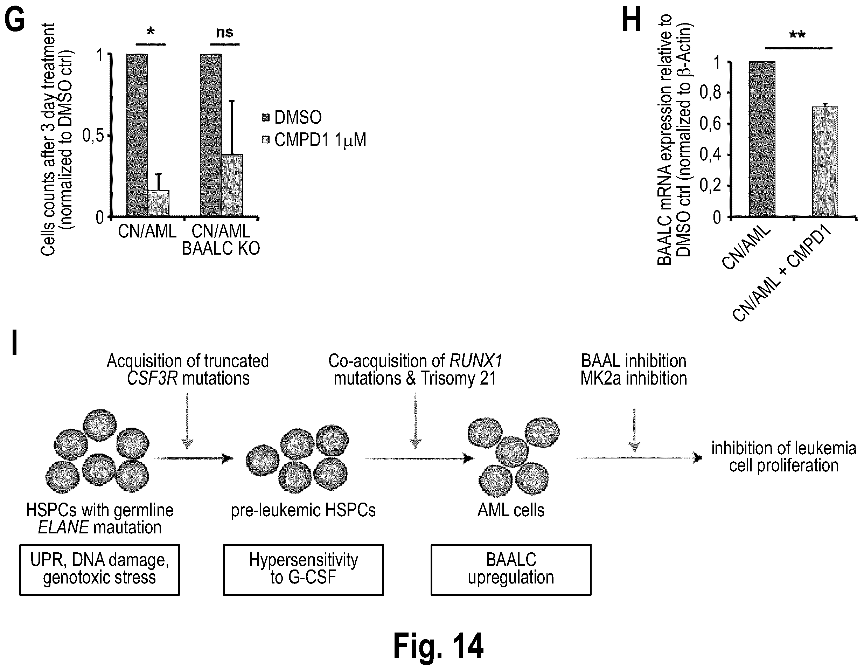

- Fig. 13:: MK2a- and p38α- inhibitor treatment of iPSC-derived CD34⁺ cells, primary CN/AML blasts and de novo AML blasts, related to Figure 14. (A) Connectivity MAP analysis of RNA-seq data of CN/AML and CN/AML BAALC KO iPSC-derived CD45⁺CD34⁺ cells. pc_selection represents the percent of total perturbagens, querying the column sample against selected rows that exceed the given thresholds. (B) Flow cytometry analysis of intracellular MK2a and p38α levels in CN, CN/AML, CMPD1-treated CN/AML (1 µM of CMPD1 for 1 day), and CN/AML BAALC KO iPSC-derived CD34⁺CD45⁺ cells. Data are represented as mean ± SD; two independent experiments of both patients. (C) Proliferation rate of Kasumi-1 WT cells treated with either AZD-6244 (1, 2 and 5 µM), CMPD1 (1, 2 and 5 µM), or DMSO for 7 days. Data are represented as mean ± SD; two independent experiments. *P < 0.05, ****P < 0.0001 compared to DMSO control. (D) Proliferation rate of Kasumi-1 WT cells treated with UO126 (1, 5 and 10 µM) or DMSO for 7 days. Data are represented as mean ± SD; two independent experiments. (E) Proliferation rate of HD, CN, and CN/AML iPSC-derived CD34⁺CD45⁺ cells treated with either 1µM AZD-6244 or DMSO for 7 days. Data are represented as mean ± SD; two independent experiments. *P < 0.05, **P < 0.01 compared to DMSO control. (F) Proliferation rate of primary CN/AML blasts treated with either 1µM AZD-6244 or DMSO for 7 days. Data are represented as mean ± SD; two independent experiments. **P < 0.01 compared to DMSO control. (G) Proliferation rate of de novo AML blasts treated with either AZD-6244 (1µM and 2µM) or DMSO for 7 days. Data are represented as mean ± SD; two independent experiments. **P < 0.01, ***P < 0.001 compared to DMSO control.
- Fig. 14:: Inhibition of proliferation of iPSC-derived and primary CN/AML or de novo AML blasts by MK2a inhibitor, CMPD1. (A) Flow cytometry analysis of intracellular phospho-MK2a and phospho-p38α protein levels in CN, CN/AML, CMPD1-treated CN/AML (1 µM CMPD1; 24 hours), and CN/AML BAALC KO iPSC-derived CD34+ cells. Data are represented as mean ± SD from two independent experiments of two CN/AML patients. * P < 0.05, **P < 0.01, ***P < 0.001 compared to CN/AML. (B) Proliferation rate of iPSC-derived CD34+ cells from HD, CN and CN/AML clones treated with either 1 µM CMPD1 or DMSO for 7 days. Data are represented as mean ± SD from two independent experiments. **P < 0.01 compared to DMSO control. (C) mRNA expression of BAALC in blasts of CN/AML patients. Gene expression levels were normalized to β-actin and shown relative to HD. Data are represented as mean ± SD from two independent experiments. **P < 0.01 compared to CD34⁺ cells of 3 HDs. (D) Proliferation rate of primary CN/AML blasts treated with 1 µM CMPD1 or DMSO for 7 days. Data are represented as mean ± SD from two independent experiments. ***P < 0.001 compared to DMSO control. (E) mRNA expression of BAALC in de novo AML blasts. Gene expression levels were normalized to β-actin and shown relative to HD. Data are represented as mean ± SD; two independent experiments. ***P < 0.001 compared to CD34+ cells of 3 HDs. (F) Proliferation rate of de novo AML blasts treated with 1 µM and 2 µM CMPD1 or DMSO for 7 days. Data are represented as mean ± SD from two independent experiments. **P < 0.01, ***P < 0.001 compared to DMSO control. (G) Proliferation rate of iPSC-derived CD34⁺ cells from CN/AML and CN/AML BAALC KO clones treated with 1 µM CMPD1 or DMSO for 7 days. Data are represented as mean ± SD from two independent experiments/patient. *P < 0.05 compared to DMSO control. (H) mRNA expression of BAALC in CN/AML iPSC-derived CD34⁺ cells treated with 1 µM CMPD1 for 24 hours. Data are represented as mean ± SD from two independent experiments/patient. **P < 0.0 normalized to DMSO control. (I) Schematic diagram of the role of BAALC upregulation in CN-related leukemia development. In CN patients with inherited CN-associated mutations (e.g., ELANE), genetically 'unfit' HSPCs acquire CSF3R mutations. The acquisition of CSF3R mutations along with prolonged exposures to high doses of G-CSF leads to the clonal selection of pre-leukemia HSPCs, which exhibit elevated proliferation and reduced differentiation upon G-CSF treatment. The co-acquisition of further RUNX1 haploinsufficiency or missense RUNX1 mutations along with trisomy 21 leads to elevated BAALC expression levels and leukemogenic transformation. Inactivation of BAALC in CN/AML cells using CRISPR/Cas9-mediated gene-editing restores normal hematopoietic differentiation, while selective inhibition of MK2a phosphorylation with CMPD1 efficiently blocks proliferation of CN/AML cells.

### EXAMPLES

### 1. Introduction

Pre-leukemia bone marrow failure syndromes are characterized by abnormal differentiation and functions of hematopoietic stem and progenitor cells (HSPCs). They frequently culminate in the development of myelodysplastic syndrome (MDS) or acute myeloid leukemia (AML). The prevailing hypothesis of leukemia development in inborn pre-leukemia syndromes postulates that homeostasis, self-renewal, proliferation or differentiation of HSPCs is disturbed owing to inherited mutations. These "unfit" HSPCs are exposed to constant stress through intrinsic HSPCs defects and extrinsic abnormalities within the HSPCs niche, which together may lead to the acquisition of mutations and/or chromosomal abnormalities in leukemia-associated genes. The background of an inherited disease can create selective pressure that supports the outgrowth and evolution of mutant HSPCs clones. Severe congenital neutropenia (CN) is a pre-leukemia syndrome that, in the majority of patients, is caused by heterogeneous *ELANE* mutations encoding neutrophil elastase (NE). The HSPCs of CN patients fail to differentiate into neutrophilic granulocytes, without any severe maturation defects in other blood lineages. The cumulative incidence of MDS or AML in CN patients is approximately 20% after 20 years. Exposure of CN-HSPCs to high concentrations of granulocyte colony-stimulating factor (G-CSF) partially reverses granulocytic maturation defects. However, there is a correlation between susceptibility to G-CSF therapy and frequency of leukemia. The inventors recently reported a high frequency of acquired cooperative mutations in CSF3R (encoding the G-CSF receptor) and *RUNX1* (runt-related transcription factor 1) in 55% of CN/AML patients (31 patients were studied). Recently, the inventors described a clinical case of two cyclic neutropenia (CyN) patients with acquired CSF3R mutations. One of these patients co-acquired *RUNX1* mutation, trisomy 21 and developed AML. In the majority of CN and CyN patients who developed AML or MDS, the emergence of HSPCs clones carrying a CSF3R mutation preceded the co-acquisition of *RUNX1* mutations in the same cells. The mechanism of leukemogenic transformation of HSPCs downstream of acquired *CSF3R* and *RUNX1* mutations on the pathological background caused by CN-associated inherited mutations is unclear. CSF3R mutations are stop-codon mutations localized in the intracellular domain of the G-CSF receptor, which is responsible for the inhibition of STAT5 mediated proliferative signals by SOCS3, and the activation of STAT3-dependent differentiation. Missense and nonsense *RUNX1* mutations in the DNA-binding Runt homology domain (RHD), or truncating mutations in the transactivation domain (TAD) have been detected in CN/AML patients. Numerous studies have reported a diversity of mechanisms underlying the pathogenesis of missense and nonsense *RUNX1* mutations. Most of these studies are performed in a mouse system or by overexpressing mutated *RUNX1.* Interestingly, *RUNX1* maps to chromosome 21, and leukemic blasts from some CN/AML patients harbor trisomy 21. Thus, the ratio of mutant to wild type *RUNX1* alleles may contribute to leukemogenesis.

The establishment of a viable experimental model would be essential for studying multistep leukemogenesis in CN. Transgenic mice with a knock-in of the human *ELANE* mutation exhibit no neutropenia phenotype under steady-state conditions. Despite some limitations, patient-specific induced pluripotent stem cells (iPSCs) are an excellent alternative to animal models. Generation of CN patient-specific iPSCs that recapitulate the maturation arrest of granulopoiesis has been previously described. iPSCs have been used to study leukemogenesis and to identify compounds targeting AML. Using CRISPR/Cas9 gene-editing, it is possible to introduce distinct mutations in iPSCs and to study stepwise, stage-specific leukemia progression. This approach allows the development of leukemia models to compare different types of mutations (e.g., missense versus nonsense) in endogenously expressed proteins (e.g., RUNX1).

In the present study, the inventors established an *in vitro* model of leukemia evolution in CN using patients-derived iPSCs and CRISPR/Cas9-mediated introduction of gene mutations. Using this model, the inventors described the upregulation of *BAALC* (brain and acute leukemia, cytoplasmic) as a key leukemogenic event. The inventors identified a small molecule inhibitor of MK2 phosphorylation that targets CN/AML blasts without affecting healthy HSPCs. This strategy could be applied to treat CN/AML or *de novo* AML patients with *RUNX1* mutations and/or elevated BAALC expression.

### 2. Material and Methods

### iPSC lines

CN and CN/AML patient samples for reprogramming were received from Severe Chronic Neutropenia International Registry (SCNIR), Hannover, Germany. Informed written consent was obtained from all participants of this study. The experiments involving human inducible pluripotent stem cells (iPSCs) were performed with the approval obtained from the Ethical Review Board of the Medical Faculty, University of Tübingen.

### Generation of iPSC

Peripheral blood mononuclear cells (PBMNCs) or bone marrow mononuclear cells (BMMNCs) from 2 male CN/AML patients at different stages of leukemic transformation and male healthy donors were reprogrammed using Oct4, Sox2 and KLF4 (OSK) lentivirus (pRRL.PPT.SF.hOct34.hKlf4.hSox2.i2dTomato.pre.FRT, kindly provided by A. Schambach, MHH Germany). Additional mutations in *CSF3R* or *RUNX1* were introduced using CRISPR/Cas9 gene-editing, if not already obtained by reprogramming. iPSCs were cultured on SNL-feeder cells (Public Health England) for general maintenance or feeder-free on Geltrex with StemFlex medium (Thermo Fisher Scientific) for further gene-editing experiments.

### Reprogramming of PBMNCs

1.5x10⁶ PBMNCs were cultured for 6 days in CD34⁺ cells expansion medium (Stemlinell medium, Sigma-Aldrich) supplemented with 10 % FCS, 1 % Pen/Strep, 1 % Glutamine and cytokines: IL-3 (20 ng/ml), II-6 (20 ng/ml), TPO (20 ng/ml), SCF (50 ng/ml) and FLT3L (50 ng/ml). All cytokines were purchased from R&D Systems. After 1 week, cells were transferred to Retronectin (Clontech)-coated 12-well plates together with OSK lentiviral supernatant at a multiplicity of infection (MOI) of 2. Four days later, cells were transferred to SNL-feeders and cultured in a 1:1 mixture of iPSC-medium and CD34⁺ cell expansion medium supplemented with 2 mM valproic acid and 50 µg/ml Vitamin C. Medium was gradually changed to iPSCs medium only. First iPSCs colonies appeared approximately three weeks after initiation of reprogramming.

### Sequencing of iPSCs

Genomic DNA of iPSCs was isolated using Nucleo-Spin Tissue Kit (Machery-Nagel) and DNA regions for sequencing were amplified using the following primers: *RUNX1*-F 5'-ACATCCCTGATGTCTGCATTTGTCC-3' [SEQ ID NO: 33], *RUNX1-*R 5'-TGTGGGTTTGTTGCCA TGAAACGTG-3' [SEQ ID NO: 34], *ELANE-F 5'-*CGCCCTGAGCCTTGGTGACG-3' [SEQ ID NO: 35], *ELANE-R 5'-*AGCCACGGTGCCTGTTGCTG-3' [SEQ ID NO: 36], CSF3R-F 5'-ATGGCATGTGTCAGGCATGT-3' [SEQ ID NO: 37], CSF3R-R 5'-AGTCACAGCGGAGATAGTGC-3' [SEQ ID NO: 38]. Sanger Sequencing was performed by GATC Biotech.

### iPSCs culture

iPSCs were maintained on mitomycin-C treated SNL-feeder cells (Public Health England) in iPSC-medium consisting of DMEM F12 (Sigma-Aldrich) supplemented with 20 % Knockout Serum Replacement (Invitrogen), 30 ng/ml bFGF (Peprotech), 1 % non-essential amino acids solution (Invitrogen), 100 µM 2-Mercapto-Ethanol and 2 mM L-Glutamine. iPSC-medium was replaced every day. For CRISPR/Cas9 gene-editing experiments and expansion of single cell derived clones, iPSC lines were cultured on Geltrex with StemFlex medium (Life Technologies).

### CRISPR/Cas9 gene-editing of iPSC lines

The sgRNAs capable of knocking out of the BAALC gene by targeting the first coding exon are listed in Table 3. Corresponding sgRNAs (Table 2) were cloned into all-in one pSpCas9(BB)-2A-GFP (PX458) plasmid, a gift from Feng Zhang (Addgene plasmid # 48138). CN- and CN/AML iPSC lines were nucleofected with 1-5 µg PX458-sgRNA construct using P3 Primary Cell 4D-Nucleofector^{™} X Kit L (Lonza) and 4D Nucleofector (Lonza) or reverse transfected with *Trans*IT^{®}-LT1 Transfection Reagent (Mirus). *BAALC* KO in HD iPSCs was introduced by transfection with cytosine base editor plasmid BAALC_p.19.NLS.BE3.2xNLS.GFP.PX458 which was generated by sticky end cloning of BE3 into PX458 backbone followed by cloning guide RNA sequence BAALC p.19 (Table 2). BE3 insert was isolated from pCMV-BE3 plasmid which was a gift from the David Liu lab (Addgene plasmid # 73021). GFP⁺ cells were sorted 48 hours post-transfection and cultured on the Geltrex. Single-cell clones were analyzed by Sanger sequencing at the gene-edited target regions. *BAALC* KO in primary CN/AML cells was achieved by nucleofection of Cas9-sgRNA-BAALC-p.20 ribonucleoprotein (RNP) complex using P3 Primary Cell 4D-Nucleofector^{™} X Kit L (Lonza) and 4D Nucleofector (Lonza). The RNP nucleofection protocol has been recently published by our group. Off-target sites were predicted using http://crispor.org. The top 3 sites with the highest off-target scores and/or exon localization were selected for Sanger sequencing (Table 4).

### EB-based hematopoietic differentiation of iPSCs

iPSCs were dissociated from SNL-feeders or Geltrex (Thermo Fisher Scientific) coated plates using PBS/EDTA (0.02%) for 5 min. EB generation was done via centrifugation of 20.000 cells per EB in 96-well plates using APEL serum-free differentiation medium (Stemcell Technologies) supplemented with bFGF (20 ng/µl) and ROCK Inhibitor Y-27632 dihydrochloride (Tocris). Next day, BMP4 (40 ng/ml) (R&D Systems) was added to the culture to induce mesodermal differentiation. On day four, EBs were plated on Matrigel-coated 6-well plates (10 EBs/well) in APEL medium supplemented with VEGF (40 ng/ml) (R&D Systems), SCF (50 ng/ml) (Peprotech) and IL-3 (50 ng/ml) (Peprotech). For neutrophilic differentiation, medium was changed 3 days later to fresh APEL medium supplemented with IL-3 (50 ng/ml) and G-CSF (50 ng/ml) (Amgen). First hematopoietic suspension cells appeared on day 12 - 14. Suspension cells were harvested every 3 - 4 days and analyzed starting from day 14 to day 32.

### Culture of iPSC-derived CD45⁺CD34⁺ cells, de novo CN/AML cells and AML cells on SL/SL feeder cells and drug treatment

1-3x10⁵ iPSC-derived CD45⁺CD34⁺ cells, primary CN/AML blasts, or de *novo* AML blasts were cultured on SL/SL feeder cells producing FLT3L (kindly provided by C. Eaves, Vancouver, Canada) in HLTM/Myelocult H5100 medium (Stemcell Technologies) supplemented with 10-⁶ M hydrocortisone, IL-3 (20 ng/ml), II-6 (20 ng/ml), TPO (20 ng/ml), SCF (50 ng/ml) and FLT3L (50 ng/ml) for 7 days with medium change every 3 - 4 days. For drug treatment, 1µM AZD-6244, 1 µM CMPD1 or DMSO was added to culture medium and incubated for 7 days with medium change and addition of fresh drugs every 3 days. After 7 days, cell viability was assessed by Trypan blue staining and counting using Neubauer cell counting chambers.

### Transduction of CD34⁺ cells with RUNX1 constructs

CD34⁺ cells from healthy donors (2×10⁵/well) were transduced with lentiviral supernatant at a MOI of 5. Lentiviral particles expressed either control plasmid, WT *RUNX1* or two *RUNX1* mutants (p.R139G and p.R174L) cloned into pRRL.PPT.SFFV.i2RFP vector (kindly provided by A. Schambach, MHH, Hannover, Germany). A second transduction was performed the next day. Seventy-two hours post-transduction, GFP-positive cells were sorted and analyzed for *RUNX1* and *BAALC* mRNA expression by qRT-PCR. Vector and primer information is available upon request.

### Quantitative RT-PCR

RNA was isolated using RNeasy Micro Kit (Qiagen). cDNA was prepared from 0.2-1 µg of total RNA using Omniscript RT Kit (Qiagen). qPCR was performed using SYBR Green qPCR master mix (Roche) and Light Cycler 480 (Roche). Data were analyzed using ddCT-method. Target genes were normalized to *GAPDH* and/or β-Actin as housekeeper genes. qPCR Primer information are available upon request.

### Western blotting

1x10⁶ cells were lysed in 200 µl of 3x Lämmli buffer. Protein was denatured for 10 min at 95 °C. 5 µl of cell lysate in the Lämmli buffer were loaded per lane. Proteins were separated on a 12% polyacrylamide gel and transferred on a nitrocellulose membrane (GE Healthcare) (1 hour, 100V, 4°C). Membrane was blocked for 1 hour in 5 % BSA/TBST and incubated with primary antibody overnight (at 4°C). The following primary antibody were used: anti-RUNX1/AML1 (Cell Signaling Technology, #4334, 1:500), anti-STAT5a (Cell Signaling Technology, #4807, 1:500) anti-G-CSFR (Santa Cruz Biotechnology, #sc-74026, 1:500), and β-Actin (Cell Signaling Technology, #4970, 1:1000) and anti-BAALC (Santa Cruz Biotechnology, #sc-515606, 1:500). Next, membranes were washed and incubated with secondary HRP-coupled antibody (Cell Signaling Technology, #7076 or #7074, 1:2000) for 1 hour at room temperature. Pierce ECL solution (Thermo Fisher Scientific) and Amersham Hyperfilm (GE Healthcare) were used to detect chemiluminescence signals of proteins.

### Alkaline Phosphatase Assay

iPSC colonies on SNL-feeders at day 10 of culture were washed with PBS, fixed in 4 % PFA /10 % sucrose in water and stained with NBT/BCIP staining dye (Sigma-Aldrich) for 20 min at RT.

### Array-CGH

Array-CGH was performed using the Agilent Human Genome Microarray Kits 2 x 400K (Agilent Technologies). Labelling and hybridization of genomic DNA was performed according to the protocol provided by Agilent. Microarray slides were scanned using an Agilent microarray scanner G2505B at a resolution of 2 µm. For image analysis, default CGH settings of Feature Extraction Software (Agilent Technologies) were applied. Output files from Feature Extraction were subsequently imported into Agilent's CGH data analysis software, Genomic-Workbench. The Aberration Algorithm ADM2 was applied and Aberration Filters were set to: threshold 7.0, at least 4 probes with mean log2 ratio of +/-0.3 leading to a resolution of approximately 20 kb.

### Flow Cytometry

30.000 suspension cells collected from EB-based hematopoietic differentiation system were used for flow cytometry. For cell surface staining, cells were prepared in PBS/1% BSA containing 0.05 % sodium azide and stained with specific mouse monoclonal anti-human antibodies. For detection of hematopoietic progenitor cells, a multicolor FACS antibody panel for 'early-stage' hematopoietic differentiation using the following antibody was applied: CD33-BV421 (BioLegend, BL), CD34-PeCy7 (BD Biosciences, BD), KDR-AF647 (BL), CD43-PE (BD), CD41a-FITC (BD), CD235a-FITC (BD), CD45-BV510 (BL), 7-AAD (BD). For detection of mature myeloid cells, a multicolor FACS antibody panel for 'late-stage' hematopoietic differentiation using the following antibody was applied: CD15-PE (BD), CD16-FITC (BD), CD14-APC-H7 (BD), CD45-BV510 (BL), CD33, BV-421 (BL), 7-AAD (BD). For iPSCs characterization, the stem cell surface markers TRA1-60-PE (eBioscience) and SSEA4-FITC (BD) were analyzed. TRA1-85-APC (R&D Systems) was used as a human iPSCs marker. Anti-mouse IgGk beads (BD) were used for compensation. Samples were analyzed using FACSCanto II (BD) and FlowJo V10 (BD).

### Morphological analysis

Wright-Giemsa stained cytospin slides were prepared using Hema-Tek slide stainer (Ames). Hematopoietic cells were classified into 4 groups according to the differentiation state: myeloblast and promyelocyte (MB/ProM), myelocyte and metamyelocyte (Myelo/Meta), band and segmented neutrophils (Band/Seg) and monocytes/macrophages (Mο/MΦ).

### Colony Forming Unit (CFU) Assay

10.000 suspension cells from EB-based iPSC hematopoietic differentiation at day 14 were used for CFU-Assay using Methocult H4435 enriched medium (StemCell Technologies). Colonies were counted after 10-14 days.

### Digital PCR (dPCR)

Digital PCR was used for absolute endpoint quantification of gene copy numbers using TaqMan SNP genotyping Assay and QuantStudio 3D Digital PCR System (Thermo Fisher Scientific). dPCR was performed according to QuantStudio 3D Digital PCR protocol using genomic DNA. Data was processed using QuantStudio 3D Digital PCR Analysis Suite Software (Thermo Fisher Scientific).

### RNA sequencing (RNA-seq)

mRNA was isolated from iPSC-derived CD45⁺CD34⁺ cells using RNeasy Mini- or Micro Kit (Qiagen). Isolated RNA was quantified (Qubit RNA assay kit, Thermo Fisher Scientific) and quality was assessed using an Agilent 2100 Bioanalyzer. Samples with high RNA integrity number (RIN > 8) were selected for library preparation. Using the TruSeq RNA Sample Prep Kit (Illumina) and 100-500 ng of total RNA for each sequencing library, poly (A) selected single- and pair-end sequencing libraries were generated according to the manufacturer's instructions. All libraries were sequenced by the c.ATG (University Hospital Tuebingen) on an Illumina NextSeq500 platform at a depth of 20-30 million reads each. Read quality of RNA-seq data in fastq files was assessed using FastQC (v0.11.4). Reads were aligned using STAR (v2.4.2a) allowing gapped alignments to account for splicing against a custom-built genome composed of the Ensembl Homo Sapiens genome v90. Alignment quality was analyzed using samtools (v1.1) and visually inspected in the Integrative Genome Viewer (v2.3.67). Read counts per gene were extracted with HTSeq count. Genes covered with less than 50 reads were excluded from the analysis leaving >12.000 genes for determining differential expression between the experimental groups. Normalized read counts for the genes were obtained in DESeq2 package (v1.8.2) and used for GSEA to find significant differences between groups. Differential expression analysis was performed using the DESeq2 package. Differentially expressed genes with log2FC > 1 or log2FC < -1, adjusted P-value < 0.05 were selected for downstream analysis. Heat maps were produced in Heatmapper. Venn diagrams were created in Venny 2.1 (https://bioinfogp.cnb.csic.es/tools/venny/index.html). eXpression2Kinases algorithm was used to identify upstream transcription factors that regulate differentially expressed genes and to perform kinase enrichment assay.

### Connectivity map (CMAP) analysis

CLUE platform (https://clue.io/cmap) was used for CMAP analysis to evaluate the gene expression profiles of CN/AML and CN/AML BAALC KO RNA-seq data sets for connectivity to known perturbagens.

### Intracellular flow cytometry for phospho-MK2a, phospho-p38, MK2a and p38 in iPSC-derived CD34⁺ cells

iPSC-derived CD45⁺CD34⁺ cells were fixed for 15 min with 4% PFA and permeabilized for 30 min with 90% Methanol. Intracellular protein staining was performed with the following antibodies for 45 min at RT: phospho-p38 PE (sc-166182), p38 AF 647 (sc-81621), p-MK2a FITC (sc-293139) and MK2a AF 647 (sc-393609). All antibodies were purchased from Santa Cruz Biotechnology. The following isotype controls were used: IgG2a, k PE (Miltenyi Biotec), IgG1k AF 647 (BD) and IgG1k FITC (BD). Samples were measured on BD FACSCanto II.

### Histone H3 trimethyl Lys27 ELISA

Total histone protein extracts were isolated from CN and CN/AML iPSC-derived HSPCs of both CN/AML patients using the EpiQuik^{™} Total Histone Extraction Kit (Epigentek, catalog # OP-0006). Yield of the total histone protein extraction was measured by Quant-iT^{™} Qubit^{™} protein assay kit (Thermo Fisher Scientific, catalog # Q33212). The inventors quantified trimethylated lysine 27 in histone H3 (H3K27me3) using the Histone H3 methylated Lys27 ELISA kit (Active Motif, catalog #53106) according to the standard protocol. The assay was performed in duplicates using 6 µg of total histone protein extract per well.

### Quantification and statistical analysis

Data are presented as mean ± standard error of the mean (SEM). Comparison of two groups was performed using unpaired t-test. Statistical analyses were performed using GraphPad Prism 8 (GraphPad Software Inc.), Microsoft Excel 2010 (Microsoft Corp.) or DESeq2. A p value <0.05 was considered significant.

### 3. Results

### Missense RUNX1 mutations are associated with trisomy 21 in CN/AML patients

A comparison of cytogenetic abnormalities and type of acquired *RUNX1* mutations in 23 CN/AML patients revealed that seven of 14 CN/AML patients with missense *RUNX1* mutations only co-acquired numerical or structural abnormalities of chromosome 21 (trisomy 21, add (21q)). In contrast, tetrasomy 21 was detected in 1 out of 5 CN/AML patients with nonsense or frameshift *RUNX1* mutations (Table 1). Digital PCR (dPCR) revealed two copies of mutated missense *RUNX1* in two CN/AML and one CyN/AML patients with trisomy 21, suggesting a crucial role of the dosage of missense *RUNX1* for leukemia initiation (Figure 1A; Figure 2A).

**Table 1**

| **Patient number** | **AML subtype** | **Karyotype** | **Inherited mutation** | **Acquired *RUNX1* mutation** | **Acquired *CSF3R* mutation** |
|---|---|---|---|---|---|
| 1 | AML M1 | 45,XY,-7;46,XY 47,XY+21;46,XY | *ELANE* (C151Y) | R139G | Q718X |
| 2 | AML M4 | 46,XY | *ELANE* (G214R) | R139Q | Q720X |
| 3 | AML FAB NA | 47,XY, 21/46, XY | *ELANE* (G174R | D171N | Q718X, Q726X |
| 4 | AML M2 | 47,XX,#21 | GPT1 | K83Q | Q720X |
| 5 | AML/B-ALL | 46,XY,add(21q) | *ELANE* (A57V) | R174L | neg |
| 6 | AML M2 | 47,XY,+21 | *ELANE* (A233P, V235TfsX) | D171N | Q741X |
| 7 | AML M1 | 46,XY-7, +21 | *ELANE* (C151 S) | R135K | Q731X |
| 8 | AML M5 | 45,XX,-7 | *ELANE* (L152P) | R80S | Q726X |
| 9 | AML FAB NA | 46, XY, t(9,11) | *ELANE* (N113K) | R64P | Q718X |
| 10 | AML FAB NA | 45,XY,-7 | *WAS* (L270P) | R80S | Y729X |
| 11 | MDS | 46,XY | *HAX1* (V44X) | I22K | neg |
| 12 | MDS | 46,XX | *HAX1* (V44X) | L29S, R64P | Y729X |
| 13 | AML M1 | 46,XY | *ELANE* (IVS+1G>T) | K83Q | Q718X |
| 14 | MDS-EV | 46,XX,dup(21)(q22.1q22.3) | *Neg* | S114P, Y380G394delinsC | Q726X |
| 15 | pre-B ALL | 48,XX,del(5)(q21q349),p21.p 22(16)/46,XX | *ELANE* (G185R) | A160T,S114X | Q702X |
| 16 | AML M1 | t(p1;q3) | *ELANE* (C151Y) | R139X | Q731X |
| 17 | MDS-EB | 46,XX,add(2)(q37),add(7)(q22) | *WAS* | Q370X | Y729X |
| 18 | AML M2 | 47,XX +mar[8], 47, idem, del(10)(q32) | *ELANE* (G214R) *GFI1* | R174X, L294Qs6 | Q739X |
| 19 | AML M0 | 45,XX,-7;46,XX | *WAS* (S478I) | Intron 4, c.415_427dup6 | Q707L |
| | | | | Intron 4, c.421_427dup7 | |
| 20 | MDS/ AML M1 | 46,XY-7, +21 | *ELANE* (S 126L) | R135K | Q726P |
| 21 | MDS-EB | 45,XY - 7, 46XY | *HAX1 (V44X)* | F13TrpfsX14, R139ProfsX47 | Q726P |
| 22 | AML FAB NA | 46.XX | *ELANE* (A79VfsX9) | R139X, V137D | neg |
| 23 | BALL/AML | 49,XXdel(5)(q13q33),+21,+21+22/47, XX-7,+21,mar1/46;XX | *ELANE* (V101M) | R139fs | Q754X |

### iPSC model of stepwise leukemogenic transformation in CN/AML

The inventors generated iPSCs from two CN/AML patients harboring *ELANE* mutations, p.C151Y and p.G214R (Figure 1B). Using mononuclear cells (MNCs) isolated from peripheral blood (PB MNCs) of CN patient 1 at time of overt AML, the inventors generated an iPSC clone carrying *ELANE* p.C151Y mutation only (CN1) and two iPSC clones carrying *ELANE* mutation and acquired nonsense CSF3R p.Q741X mutation in combination with missense *RUNX1* p.R139G mutation and trisomy 21 (CN/AML1.1 and 1.2) (Figures 3A and 3B). dPCR showed one copy of WT and two copies of mutated *RUNX1* in iPSCs and CD45⁺ cells (Figure 1C), in line with observations in primary CN/AML blasts. Using CRISPR/Cas9 gene-editing (Table 2), the inventors generated two iPSC lines, one carrying the heterozygous CSF3R mutation p.Arg734SerfsX35 (CN1+CSF3R R734fs) and another carrying the compound heterozygous CSF3R mutations p.Gln741AsnfsX59 and p.Ser742PhefsX29 (CN1+CSF3R Q741fs) (Figure 1B; Figures 3C and 3D).

**Table 2**

| **sgnNA name** | **sgnNA sequence** | **On-target score¹** | **Specificity score²** | **Out of frame score³** | **Number of off-targets for 0-1-2-3-4 mismatches, total number of mismatches** | **SEQ ID NO:** |
|---|---|---|---|---|---|---|
| *BAALC* p.14 | GTCCAGCTCTCGTAGTAGCG | 70 | 99 | 67 | 0 - 0 - 0 - 0 - 13, 13 off-targets | 1 |
| *RUNX1* p.20 | CATCTTGCCTGGGCTCAGCG | 69 | 68 | 62 | 0 - 0 - 3 - 36 - 234, 273 off-targets | 3 |
| *RUNX1* p.R174 | CACTTACTTCGAGGTTCTCG | 61 | 47 | 68 | 0 - 0 - 0 - 2 - 33, 35 off-targets | 4 |
| CSF3R p.Q741 | CTGGTGCCAGACTGGGATTG | 50.7 | 66 | 64 | 0 - 0 - 2 - 26 - 229, 257 off-targets | 5 |
| CSF3R p.R734 | GCTGGGTGGAAACTGCTCTT | 33.2 | 77 | 71 | 0 - 0 - 1 - 14 - 142, 157 off-targets | 6 |
| *CD109 p.14* | GCGCGGCGGTGCACACGCAG | 62 | 86 | 65 | 0 - 0 - 0 - 9 - 49, 58 off-targets | 7 |
| *HPGDS* p.2 | AGTGAGTTTGTAGTTTGGCA | 60.3 | 67 | 65 | 0 - 0 - 0 - 23 - 200, 223 off-targets | 8 |
| *BAALC* p.19 | GGGTCCAGCTCTCGTAGTAG | 54.9 | 96 | 64 | 0 - 0 - 0 - 3 - 52, 55 off-targets | 2 |

| | | | | | | |
|---|---|---|---|---|---|---|
| ¹ Score is from 0-100. Higher is better. ² Score is from 0-100. Higher is better. ³ Score is from 0-100. Higher is better. | | | | | | |

Table 3 lists all sgRNA which are capable of knocking out the BAALC gene.

**Table 3**

| **Human genome reference** | **Chr** | **Start position** | **Strand** | **Guide RNA sequence** | **PAM sequence** | **SEQ ID NO:** |
|---|---|---|---|---|---|---|
| GRCh38 | Chr8 | 103140949 | 1 | AGCCCCGCTACTACGAGAGC | TGG | 39 |
| GRCh38 | Chr8 | 103140989 | -1 | CGGCGCGTCCGAGTCGGTGT | AGG | 40 |
| GRCh38 | Chr8 | 103140942 | -1 | GGGTCCAGCTCTCGTAGTAG | CGG | 2 |
| GRCh38 | Chr8 | 103140940 | -1 | GTCCAGCTCTCGTAGTAGCG | GGG | 1 |
| GRCh38 | Chr8 | 103140995 | -1 | GCTGGGCGGCGCGTCCGAGT | CGG | 41 |
| GRCh38 | Chr8 | 103140932 | -1 | CTCGTAGTAGCGGGGCTCGA | TGG | 42 |
| GRCh38 | Chr8 | 103141039 | -1 | GAGTGCAGGCCGCCCGCTTC | GGG | 43 |
| GRCh38 | Chr8 | 103140992 | 1 | CTGGCTCACCTACACCGACT | CGG | 44 |
| GRCh38 | Chr8 | 103141053 | -1 | CCGGCCACTTACCCGAGTGC | AGG | 45 |
| GRCh38 | Chr8 | 103141040 | -1 | CGAGTGCAGGCCGCCCGCTT | CGG | 46 |
| GRCh38 | Chr8 | 103140955 | 1 | GCTACTACGAGAGCTGGACC | CGG | 47 |
| GRCh38 | Chr8 | 103140941 | -1 | GGTCCAGCTCTCGTAGTAGC | GGG | 48 |
| GRCh38 | Chr8 | 103140921 | -1 | GGGGCTCGATGGCATCCGCC | CGG | 49 |
| GRCh38 | Chr8 | 103141038 | -1 | AGTGCAGGCCGCCCGCTTCG | GGG | 50 |
| GRCh38 | Chr8 | 103141052 | 1 | CGAAGCGGGCGGCCTGCACT | CGG | 51 |
| GRCh38 | Chr8 | 103141026 | 1 | AGCGCCGCCGCCCCGGACAG | CGG | 52 |
| GRCh38 | Chr8 | 103140956 | 1 | CTACTACGAGAGCTGGACCC | GGG | 53 |
| GRCh38 | Chr8 | 103140980 | -1 | CGAGTCGGTGTAGGTGAGCC | AGG | 54 |
| GRCh38 | Chr8 | 103141025 | -1 | CGCTTCGGGGCCGCTGTCCG | GGG | 55 |
| GRCh38 | Chr8 | 103141053 | 1 | GAAGCGGGCGGCCTGCACTC | GGG | 56 |
| GRCh38 | Chr8 | 103141027 | -1 | CCCGCTTCGGGGCCGCTGTC | CGG | 57 |
| GRCh38 | Chr8 | 103141038 | 1 | CCGGACAGCGGCCCCGAAGC | GGG | 58 |
| GRCh38 | Chr8 | 103140973 | 1 | CCCGGGAGACAGAATCCACC | TGG | 59 |
| GRCh38 | Chr8 | 103141064 | 1 | CCTGCACTCGGGTAAGTGGC | CGG | 60 |
| GRCh38 | Chr8 | 103141026 | -1 | CCGCTTCGGGGCCGCTGTCC | GGG | 61 |
| GRCh38 | Chr8 | 103141060 | 1 | GCGGCCTGCACTCGGGTAAG | TGG | 62 |
| GRCh38 | Chr8 | 103141037 | 1 | CCCGGACAGCGGCCCCGAAG | CGG | 63 |
| GRCh38 | Chr8 | 103141041 | 1 | GACAGCGGCCCCGAAGCGGG | CGG | 64 |
| GRCh38 | Chr8 | 103141022 | -1 | TTCGGGGCCGCTGTCCGGGG | CGG | 65 |
| GRCh38 | Chr8 | 103141012 | -1 | CTGTCCGGGGCGGCGGCGCT | GGG | 66 |
| GRCh38 | Chr8 | 103140977 | -1 | GTCGGTGTAGGTGAGCCAGG | TGG | 67 |
| GRCh38 | Chr8 | 103141013 | -1 | GCTGTCCGGGGCGGCGGCGC | TGG | 68 |
| GRCh38 | Chr8 | 103140963 | -1 | GCCAGGTGGATTCTGTCTCC | CGG | 69 |
| GRCh38 | Chr8 | 103140962 | -1 | CCAGGTGGATTCTGTCTCCC | GGG | 70 |
| GRCh38 | Chr8 | 103141019 | 1 | GCCGCCCAGCGCCGCCGCCC | CGG | 71 |
| GRCh38 | Chr8 | 103140914 | 1 | GATGGGCTGCGGCGGGAGCC | GGG | 72 |
| GRCh38 | Chr8 | 103140907 | 1 | GCAGGAGGATGGGCTGCGGC | GGG | 73 |
| GRCh38 | Chr8 | 103141009 | -1 | TCCGGGGCGGCGGCGCTGGG | CGG | 74 |
| GRCh38 | Chr8 | 103141019 | -1 | GGGGCCGCTGTCCGGGGCGG | CGG | 75 |
| GRCh38 | Chr8 | 103140906 | 1 | CGCAGGAGGATGGGCTGCGG | CGG | 76 |
| GRCh38 | Chr8 | 103140913 | 1 | GGATGGGCTGCGGCGGGAGC | CGG | 77 |
| GRCh38 | Chr8 | 103140917 | 1 | GGGCTGCGGCGGGAGCCGGG | CGG | 78 |

iPSC clones of CN patient 2 carrying *ELANE* p.G214R mutation only (CN2) or *ELANE* mutation and acquired CSF3R p.Q743X mutation (CN2+CSF3R Q743X^{+/-}) were generated from BM MNCs isolated at AML stage (Figure 1B; Figure 3A). Heterozygous frame-shift *RUNX1* p.Glu175SerfsX7 mutation was introduced in CN2+CSF3R Q743X^{+/-} iPSC clone using CRISPR/Cas9 (CN/AML2) (Figure 1B; Figure 3E).

The inventors observed no off-target effects in the CRISPR/Cas9 gene-edited iPSCs (Table 4). Array-CGH confirmed no chromosomal abnormalities in CN1 iPSC clone, trisomy 21 in CN/AML1.1 and 1.2 iPSC clones, and the additional gain of a part of chromosome 12 in CN/AML1.1 iPSC clone (Figure 3B). The chromosome 12 gain is a frequent finding during iPSC maintenance, reflecting high proliferation of these cells. No chromosomal abnormalities were detected in iPSC clones derived from CN patient 2 (Figure 3B).

**Table 4**

| **sgRNA name** | **iPSC lines tested for off-targets** | **Off-target sites selected for inspection by Sanger sequencing** | **Off-target site sequence** | **Positions of the mismatches on the off-target site** | **Off-target site sequencing results** | **SEQ ID NO:** |
|---|---|---|---|---|---|---|
| ***BAALC* p.14** | CN1 *BAALC* KO, CN2 *BAALC* KO, CN/AML 1.2 *BAALC* KO, CN/AML2 *BAALC* KO C1, CN/AML2 *BAALC* KO C2 | 4:intergenc:RP11-672A2.1-RP11-2IL23.4 | CTCCAGCCCTCACAGTAGCGAGG | *......*...**...... | negative | 9 |
| | | 4:intergenic:RNA5SP358-RNA5SP359 | GGCCAGCTCTAGTGGCAGCGCGG | .*.......*.**... | negative | 10 |
| | | 4:intron:ERC2 | TTCCTGCTCTCTTAGTAGCTAGG | * ...............* | negative | 11 |
| ***BAALC p.19*** | HD *BAAL* KO | intron:EPAS1 | GGGTCCAGCTATCTTACTAGTGG | ..........*..*..*.. | negative | 12 |
| | | intergenic:SERPINA7-RP11-506L11.1 | GTTTCCAGCTCTAGTAGTAGTAG | .**........*...... | negative | 13 |
| | | intron:HECW2 | GGGTCCAGCTCTCCTTGGAGAGG | .........*.*.*.. | negative | 14 |
| ***RUNXI* p.20** | CN/AML 1.2 *RUNXI*/KO | 2:intergenic:AFF2-IT1-AFF2 | CATCCTGCCTGGGCTCAGCTGGG | ....*..............* | negative | 15 |
| | | 3:intergenic:RP11-28211.2-GPR26 | CATTTTGCCAAGGCTCAGCGTGG | ..*....**...... | negative | 16 |
| | | 3:intron:SORCS2 | GATCTTGTCTGGGCTCAGCTGGG | * ................ . * | negative | 17 |
| ***RUNXI* p.R174** | CN/AML2 | 4:exon:RP11-244N9.4 | TATTTACTCTGAGGTTCTCGCGG | **....**......... | negative | 18 |
| | | 4:exon:MAP4K4 | CACTTACTATGTAGTTCTCGTGG | ........**.**...... | negative | 19 |
| | | 4:exon:RUNX2 | TACTTACTTCTGGGTTCCCGAGG | *.........**....*.. | negative | 20 |
| ***CSF3R p.Q741*** | CN1+*CSF3R* Q741fs | 2:intron:C8B | CAGTTGCCAGACTGGGATTGAGG | .*.*............... | negative | 21 |
| | | 3:intron:LOXHD1 | CTGGCGCCTGACTAGGATTGAGG | ......*.*..*.... | negative | 22 |
| | | 3:intron:GRHL2 | CAGGTGAAAGACTGGGATTGAAG | .*....**............ | negative | 23 |
| ***CSFR p.R734*** | CN1-*CSF3R* R7341fs | 3:intron:ILI15 | GCTGGGTGTAAGCTGCCCTTAGG | ........*.......**. | negative | 24 |
| | | 2:intron:PDE4D | GATGGCTGGAAACTGCTCCTGGG | .*.......*.**.... | negative | 25 |
| | | 3:exon:MRGPRF | GATGGCTGGAAACTGCTCCTGGG | .*.......*.**...*. | negative | 26 |
| ***CD109 p.14*** | CN/AML 1.2 *CD109* KO | 3:exon:CRIM1 | CTGCGGGGTTGCACACGCAGCGG | **....*.*.......... | negative | 27 |
| | | 4:intergenic:NKX2-5-Y_RNA | AGGAGGCAGTGCACACGCAGAGG | **....**............ | negative | 28 |
| | | 3:intergenic:EFNA2-RPS15P9 | GCGGGGCGGGGCACACACAGGGG | ...*.....*.......*.... | negative | 29 |
| ***HPGDS p.2*** | CN/AML1.2 *HPGDS* KO | 4:intron:BLMH | AGTAAGCCTGTAGTTAGGCATGG | .....***......*... | negative | 30 |
| | | 3:intron:SERPINI1 | AGTGAGTATTTAGTTTGGCTGGG | ........*.*........* | negative | 31 |
| | | 3:intron:NUMBL | TGTGTGTTTGTGGTTTGGCAGGG | .*.......*.**... | negative | 32 |

All iPSC lines expressed pluripotent stem cell markers (Figures 4A-4C), displayed alkaline phosphatase activity (Figures 4D and 4E), differentiated into three germ layers (data not shown) and had inactivated lentiviral plasmid used for reprogramming (Figures 4A and 4B).

### Impaired differentiation and elevated proliferation of CN/AML iPSC-derived HSPCs

The inventors performed *in vitro* embryoid body (EB)-based hematopoietic and myeloid differentiation in generated iPSC lines (Figure 5A). The proportions of immature cells showed only slight differences between CN1 and CN/AML1 iPSC lines (Figure 6A; Figure 5B). For CN/AML2, the percentages of CD45⁺CD34⁺ and CD34⁺CD43⁺ cells, but not of CD41a⁺CD235a⁺CD45⁻ cells, were increased as compared to CN2 (Figure 6B; Figure 5C).

To assess proliferation, HSPCs were collected at day 14 of EB differentiation and cultured on FLT3-L-secreting feeder cells. The inventors observed a substantial increase in proliferation of CN/AML iPSC-derived HSPCs from both patients as compared to CN or healthy donor (HD) HSPCs (Figures 6C and 6D). Proliferation of HSPCs expressing mutated CSF3R was significantly higher than in CN cells, but much lower than in CN/AML cells (Figures 6C and 6D). Granulocytic differentiation was markedly reduced in CN- and CN-CSF3R iPSCs compared with HD iPSCs, and practically abolished in CN/AML iPSCs (Figures 6E and 6F). Morphological analysis of cytospin preparations revealed almost no mature granulocytes in CN/AML samples (Figures 6G and 6H). CD34⁺ cells derived from CN iPSCs produced fewer numbers of CFU-Gs and CFU-GMs colonies as compared to HD iPSCs. These numbers were further decreased in CSF3R-mutated CN iPSCs and CN/AML iPSCs (Figures 6I and 6J).

### AML-associated gene expression signature in CN/AML iPSC-derived HSPCs

The inventors next examined whether top genes that they found to be upregulated in primary CN/AML blasts (*BAALC* (brain and acute leukemia, cytoplasmic), *HPGDS* (hematopoietic prostaglandin D synthase), *CD34* and *CD109* (own unpublished observations of transcriptome studies) might be also elevated in CN/AML iPSCs-derived HSPCs. The inventors confirmed increased mRNA levels of *BAALC, HPGDS, CD34* and *CD109* in primary blasts of five CN/AML patients carrying *RUNX1* and CSF3R mutations compared with CD34⁺CD33⁺ BM cells isolated from three CN patients prior to leukemia development (Figure 7A). *BAALC, CD34, HPGDS* and *CD109* expression was also increased in CD34⁺ cells derived from CN/AML1.1 and 1.2 iPSC lines compared with CN1 iPSCs, and *BAALC* mRNA was higher in CN/AML2 HSPCs as compared to CN2 cells (Figures 7B and 7C). BAALC protein was elevated in CN/AML HSPCs of both patients (Figure 7D).

The inventors further tested whether elevated *BAALC* level is caused by mutated *RUNX1.* Indeed, introduction of mutated *RUNX1* (p.R139G or p.R174L) in healthy individuals CD34⁺ cells led to markedly increased BAALC mRNA, as compared to WT *RUNX1* transduced cells (Figure 7E).

The inventors have reported elevated levels of STAT5a in primary CN/AML blasts. Here, upregulated STAT5a mRNA and protein were detected in CN/AML iPSC-derived HSPCs (Figures 7F-7I). Interestingly, *RUNX1* mRNA and protein expression was markedly increased in CN/AML1 cells with missense *RUNX1* and trisomy 21, whereas in CN/AML2, with truncated *RUNX1,* only *RUNX1* mRNA was elevated. G-CSFR protein expression was enhanced in CN/AML1, but not in CN/AML2 (Figures 7F-7I).

### Leukemogenic effects of elevated BAALC in CN

To determine which of the candidate genes, *BAALC, HPGDS, CD109* or *RUNX1,* is responsible for the leukemogenic transformation in CN, the inventors generated knockout CN/AML1 iPSCs for each of these genes (Figure 8A). The inventors created sgRNAs targeting the first coding exons of each gene, cloned them into a pSp-Cas9(BB)-2A-GFP (PX458) construct (Table 2), and generated single-cell derived CN/AML1 iPSC clones with homozygous (*RUNX1, BAALC*), or compound heterozygous *(CD109, HPDGS)* mutations (Figures 9A-9F). Pluripotency of selected iPSC clones was unaffected (data not shown), and there was no evidence of CRISPR-Cas9 off-target activity (Table 3).

*BAALC,* but not *CD109, RUNX1* or *HPGDS* knockout resulted in a dramatic induction of granulocytic differentiation and a significant reduction in proliferation of CN/AML1 iPSC-derived HSPCs (Figures 8B-8E; Figure 9G). Similarly, the inventors observed a striking increase in granulocytic differentiation along with a marked reduction in proliferation in CN/AML2 iPSC-derived HSPCs after *BAALC* KO (Figures 8F-8I; Figure 9H). In contrast, *BAALC* KO did not affect granulocytic differentiation in HD iPSCs, but induced granulopoiesis in CN iPSCs (Figure 8J, Figures 9I-9K).

The inventors also assessed the inhibitory effect of *BAALC* KO on proliferation of primary blasts from three CN/AML patients. *BAALC* KO (CRISPR/Cas9 KO efficiency was around 80 %) led to a marked reduction of cell proliferation measured on day 14 of culture as compared to mock electroporated cells (Figure 8K).

### Leukemia-specific gene expression signature in CN/AML iPSCs-derived HSPCs

The inventors compared the transcriptome of CN and CN/AML iPSCs-derived HSPCs from both patients. Differential gene expression analyses using DESeq2 R package identified 132 up- and 570 down-regulated genes, as well as 570 up- and 1422 down-regulated genes between CN/AML and CN stages for patient 1 and patient 2, respectively (log2FC > 1 or < -1, adj. P-value < 0.05; Figures 10A and 10B; Figures 11A and 11B; Tables 4 and 5). Gene set enrichment analysis (GSEA) revealed E2F targets, MYC targets and oxidative phosphorylation pathway as top significant altered pathways in CN/AML1, whereas platelet-specific genes were enriched in CN1 (Figure 10C). G2M checkpoint signaling, E2F targets and TGFβ signaling were significantly deregulated in CN/AML2, whereas the structural integrity of the ribosomes was affected in CN2 (Figure 10D).

To predict transcription factors that control differentially expressed genes, the inventors performed eXpression2Kinases analysis. The inventors detected *RUNX1* binding motifs, *GATA1, GATA2* motifs, as well as AML-associated *SUZ12* and *EZH2* motifs (Figure 10E) to be significantly enriched in CN/AML cells. *TRIM28* and *TP53* motifs were specifically enriched in CN/AML1, whereas *CEBPB, NANOG, KLF4* in CN/AML2 (Figure 10E).

Using kinase enrichment analysis (KEA), the inventors predicted kinases that phosphorylate proteins regulated by the subnetwork of prioritized transcription factors. A majority of enriched kinases, including HIPK2, MAPK1/3/14, CSNK2A1, ERK1 and AKT1, were shared between both patients at the selected threshold (P-value <10⁻⁸) (Figure 10F). Unique enriched kinases (e.g., CDK1, JNK1, ERK2) were detected in CN/AML2 only (Figure 10F).

To determine the effects of *SUZ12* and *EZH2* differential binding in CN/AML, th einventors measured total levels of H3K27me3 in CN and CN/AML iPSC-derived CD34⁺ cells. *SUZ12* and *EZH2* belong to a core subunit of the (polycomb repressive complex 2) PRC2 complex responsible for H3K27 methylation. The inventors detected reduced H3K27me3 levels in CN/AML cells compared to CN cells (Figure 11C), suggesting a modulatory effect of *RUNX1* mutations on the H3K27me3 status in CN/AML.

### BAALC-dependent leukemogenic pathways in CN/AML

To identify BAALC-dependent leukemia-associated gene expression, the inventors compared the transcriptomes of CN/AML iPSCs before and after *BAALC* KO. The inventors identified 165 up- and 254 down-regulated genes between CN/AML1 and CN/AML1 *BAALC* KO, as well as 185 up- and 381 down-regulated genes between CN/AML2 and CN/AML2 *BAALC* KO (log2FC > 1 or < -1, adj. P-value < 0.05) (Figures 12A and 12B; Figures 11D and 11E; Tables 6 and 7). *BAALC* KO led to a dramatic shift in the gene expression signature (Figure 12C,D). GSEA revealed enrichment of oxidative phosphorylation, tricarboxylic acid cycle (TCA), p53 signaling and inhibition of platelet-specific genes in CN/AML1 sample as compared to *BAALC* KO (Figure 12E). E2F targets, G2M checkpoint-associated genes, TGFβ signaling and MYC targets were enriched in CN/AML2 cells (Figure 12F).

Transcription factor enrichment analysis (TFEA) of differentially expressed genes revealed *RUNX1, GATA1*/*2, SUZ12* transcription factor binding motifs to be BAALC dependent in both patients. *AR, TCF3, RAD21, NANOG* motifs were deregulated in CN/AML1 only. *EGR1, STAT3* and *ZC3H11A* motifs were CN/AML2 specific (Figure 12G).

CSNK2A1, CDK1, GSK3B, HIPK2, MAPK14/p38α were among the top BAALC-dependent kinases in both patients (Figure 12H). CN/AML2, but not CN/AML1, showed patient-specific kinases enrichment (Figure 12H).

Key transcription factor motifs such as *RUNX1, GATA1*/*2, SUZ12, SMAD4* as well as 7 out of 12 kinases (e.g. MAPK14, MAPK1, ERK1, AKT1) were enriched in CN/AML compared to either CN or CN/AML *BAALC* KO stages in both patients (Figures 12I and 12J). These data indicate that *BAALC* KO at least partially restores CN phenotype in CN/AML cells irrespective of the *RUNX1* mutation type.

### The anti-proliferative effect of a selective inhibition of MAPK14/p38α-mediated MK2a phosphorylation in CN/AML

Using Connectivity Map (CMAP) analysis of differentially expressed genes between CN/AML and CN/AML *BAALC* KO HSPCs, we evaluated drug candidates inducing a gene expression profile similar to the *BAALC* KO. A selective inhibitor of p38α-mediated MK2a phosphorylation, CMPD1, was the first hit (Figure 13A). This result is in line with the kinase enrichment analysis - MAPK14/p38α was among the top BAALC-dependent kinases in CN/AML (Figures 10F and 12H).

The inventors assessed MK2a and p38α phosphorylation levels in CN and CN/AML iPSC-derived HSPCs as well as in CN/AML cells treated with CMPD1 and CN/AML *BAALC* KO cells. The detected elevated phospho-MK2a, but not phospho-p38α levels in CN/AML cells as compared to CN (Figure 14A). At the same time, CMPD1 treatment or *BAALC* KO resulted in a reduction of MK2a but not p38α phosphorylation in CN/AML (Figure 14A). Basal levels of non-phosphorylated MK2a and p38α proteins remained unchanged in all groups (Figure 13B). These data confirm BAALC-dependent phosphorylation of MK2a in CN/AML.

The inventors next tested the effects of CMPD1-dependent reduction of MK2a phosphorylation on the growth of CN/AML cells. The inventors treated BAALC^{high} AML cell line Kasumi-1 with CMPD1 and found markedly reduced cell proliferation as compared to DMSO control (Figure 13C). Importantly, CMPD1 treatment of CN/AML iPSC-derived HSPCs from both patients significantly reduced cell growth without affecting the proliferation of HD and CN iPSC-derived HSPCs (Figure 14B), as compared to DMSO groups. Moreover, treatment of primary blasts from three BAALC^{high} CN/AML patients with CMPD1 led to a dramatic suppression of proliferation, without affecting HD cells (Figures 14C and 14D). A less prominent effect of CMPD1 was observed in *de novo BAALC*^{high} AML samples (Figures 14E and 14F). *BAALC* KO partially protected CN/AML cells from CMPD1-induced death (Figure 14G). Additionally, BAALC mRNA levels were slightly but significantly reduced after CMPD1 treatment (Figure 14H), suggesting a feedback regulation of BAALC expression by MK2a.

It has been reported that MEK1/2 inhibitor U0126, in combination with KLF4 activation, reduces the growth of BAALC-expressing *de novo* AML cells. The inventors thus compared the effects of two MEK inhibitors, U0126 and AZD-6244, on the proliferation of CN/AML and *BAALC*^{high} *de novo* AML cells. U0126 treatment had no effects on cell proliferation (Figure 13D), whereas AZD-6244 had a moderate effect (Figures 13E-13G). No effect of AZD-6244 was observed in Kasumi cells (Figure 13C). KLF-4 activator, APTO-253, in combination with 1 µM of AZD-6244 was either not effective in a dose of 10 nM of APTO-253, or was toxic at the 100 nM and 1000 nM concentrations (data not shown).

### 4. Discussion

The inventors present here an *in vitro* model of stepwise leukemia development in pre-leukemia bone marrow failure syndromes, exemplified by CN. Using CRISPR/Cas9 gene-editing, the inventors introduced gene alterations associated with pre-leukemia and leukemia conditions in iPSC lines of CN patients. By generating iPSCs recapitulating non-leukemic and leukemia conditions, the inventors were able to evaluate hematopoietic differentiation and intracellular signaling differences between these two conditions. The inventors observed almost completely abrogated myeloid differentiation and elevated proliferation of CN/AML iPSCs compared with control or CN iPSCs. The elevated levels of AML-associated genes (e.g., *BAALC, STAT5*) in HSPCs derived from CN/AML iPSCs along with their increased proliferation and diminished differentiation argues for their leukemic features. Therefore, the *in vitro* model recapitulates step-wise leukemia development in CN. Given the absence of animal models of *ELANE-CN, in vitro* modeling of CN and leukemia using CRISPR/Cas9 gene-editing of patient-derived iPSCs is of high importance.

Different *RUNX1* mutations types, missense, nonsense, frame-shift, were detected in CN/AML. CRISPR/Cas9 gene-editing allows a comparison of the effects of different mutations in endogenously expressed proteins. The inventors provided evidence for dose-dependent outcomes of missense mutations versus *RUNX1* haploinsufficiency: missense *RUNX1* mutations, but not nonsense or frame-shift mutation in RHD, require trisomy 21 to amplify the leukemogenic effect of the mutated *RUNX1.* The inventors generated iPSC lines carrying *ELANE, CSF3R* and, additionally, mutations and chromosomal abnormalities mimicking CN/AML phenotype: (1) missense *RUNX1* mutation and trisomy 21 with two copies of mutated *RUNX1,* or (2) *RUNX1* haploinsufficiency without trisomy 21. In both conditions, myeloid differentiation was severely abrogated, and proliferation was elevated. The inventors identified signaling pathways deregulated by both missense and frame-shift *RUNX1* mutations in CN/AML cells or affected explicitly by each mutation. The vast majority of crucial signaling leukemia-causing pathways, such as the activation of the *E2F* pathway, an enrichment of *RUNX1, GATA1*/*2, SUZ12, EZH2* targets, and targets of MAPK1/3/14, HIPK2, AKT1, ERK1 kinases were overlapped in CD34⁺ CN/AML cells with missense or frameshift *RUNX1* mutations. Interestingly, some transcription factors and signaling pathways were regulated on the *RUNX1* mutation type-specific manner: missense p.R139G *RUNX1* regulated MYC targets and oxidative phosphorylation, while truncated *RUNX1* affected the constituents of ribosomes, G2M checkpoint genes, and TGF⊐ signaling. These data provide a better understanding of the mechanistic outcomes of different types of *RUNX1* mutations in leukemogenesis. Particularly interesting is the fact of the differential activity of SUZ12 and EZH2 in CN/AML vs CN. SUZ12 and EZH2 are core PRC2 subunits, suggesting possible changes of H3K27me3 distribution in *RUNX1*-mutant cells. There is an enrichment of EZH2 mutations in *RUNX1*-mutant AML and H3K27 mutations cooperate with *RUNX1* mutations in AML. In line with this, the inventors detected reduced H3K27me3 levels in CN/AML samples compared to CN samples. It would be interesting to further investigate a specific H3K27me3 distribution in CN/AML cells.

The inventors identified elevated BAALC levels in CN/AML blasts, and CRISPR/Cas9-mediated *BAALC* knockout inhibited proliferation, simultaneously inducing myeloid differentiation of CN/AML cells. *BAALC* is upregulated in RUNX1-mutated de *novo* AML and the inventors found that mutated *RUNX1* induced BAALC expression in HD HSPCs. High BAALC expression is associated with aggressive AML and poor prognosis. To study the mechanism of leukemogenesis downstream of *BAALC* activation, the inventors performed RNA sequencing of CD34⁺ cells derived from CN/AML and CN/AML *BAALC* KO cells. *BAALC* KO led to a marked shift in gene expression in CN/AML cells independent of the *RUNX1* mutation type. The inventors detected oxidative phosphorylation, MYC-, TGFβ-, E2F- and G2M checkpoint- controlling pathways to be BAALC-dependent in CN/AML. Moreover, *RUNX1, SUZ12, SOX2, GATA1*/*2, SMAD4,* and *SALL4* transcription factors as well as MAPK14, GSK3B, CSNK2A1, CDK1/2 kinases pathways were found to be downstream of *BAALC* in both CN/AML patients. These findings clearly demonstrate the fundamental role of *BAALC* in leukemia development downstream of missense and truncated *RUNX1* mutations.

Searching for the translational applications of our findings, the inventors identified the selective MK2a inhibitor, CMPD1, as the best drug candidate mimicking *BAALC* KO expression signature, when comparing CN/AML with CN/AML *BAALC* KO using CMAP. CMPD1 treatment effectively reduced proliferation of iPSC-derived and primary CN/AML blasts without affecting HD and CN HSPCs. This result was in line with inhibition of MK2a but not p38α phosphorylation by CMPD1 treatment or *BAALC* KO. These data argue for a BAALC-dependent activation of MK2a phosphorylation in CN/AML. Thus, CMPD1, or alternative MK2a inhibitors, might be implemented in the treatment of CN/AML and *BAALC*^{high} *de novo* AML. CN/AML and *BAALC*^{high} *de novo* AML patients have aggressive leukemia with poor prognosis, and our findings represent a first step toward the establishment of advanced therapies for these leukemias. Of note, CMPD1 treatment was partially effective in CN/AML *BAALC* KO cells. At the same time, BAALC expression was reduced upon CMPD1 treatment, suggesting a feedback regulation of BAALC expression by MK2a pathway.

In summary, iPSC-based *in vitro* model is reliable for investigating step-wise leukemogenesis in pre-leukemia bone marrow failure syndromes. Implementation of this model led to the characterization of high BAALC expression and elevated MK2a phosphorylation, as ultimate leukemia-causing events in CN/AML, and the identification of CMPD1 as a potential therapeutic drug for CN/AML and *BAALC*^{high} *de novo* AML patients. Inhibition of BAALC or treatment with MK2a inhibitors might prevent leukemia development in CN/AML and eliminate *RUNX1-*mutated BAALC^{high} *de novo* AML blasts (Figure 14I).

### 5. Conclusion

The inventors provide a new compound and method for the prophylaxis and/or treatment of leukemia and/or the development of leukemia, in particular AML.

## Claims

1. An inhibitor of 'mitogen-activated protein kinase-activated protein kinase 2' (MK2 inhibitor) for use in the prophylaxis and/or treatment of leukemia and/or the development of leukemia.

2. The MK2 inhibitor of claim 1, **characterized in that** said leukemia is acute myeloid leukemia (AML).

3. The MK2 inhibitor of claim 2, **characterized in that** said AML is *de novo* AML.

4. The MK2 inhibitor of claim 2 or 3, **characterized in that** said AML is AML with high expression of BAALC (BAALC^{high}).

5. The MK2 inhibitor of claim 1, **characterized in that** said development of leukemia results from a pre-existing condition that is selected from the group consisting of: congenital neutropenia (CN), familial platelet disorder with predisposition to acute myelogenous leukemia (FPD/AML) or myelodysplastic syndrome (MDS).

6. The MK2 inhibitor of any of the preceding claims, **characterized in that** it is an MK2a inhibitor.

7. The MK2 inhibitor of any of the preceding claims, **characterized in that** it is CMPD-1.

8. A pharmaceutical composition for the prophylaxis and/or treatment of leukemia and/or the development of leukemia, comprising the MK2 inhibitor of any of the preceding claims and a pharmaceutically acceptable carrier.

9. The pharmaceutical composition of claim 8, **characterized in that** the MK2 inhibitor is present per dosage unit at a concentration which, upon administration into a living being, results in a concentration in said living being which inhibits the leukemogenesis and/or eliminates leukemia cells in said living being.

10. A method for the prophylaxis and/or treatment of leukemia and/or the development of leukemia in a living being, comprising a step of inhibiting the leukemogenesis in said living being, wherein said inhibition of the leukemogenesis and/or elimination of leukemia cells is realized via the administration of an MK2 inhibitor to said living being.

11. A method for the prophylaxis and/or treatment of leukemia and/or the development of leukemia in a living being, comprising a step of inhibiting the leukemogenesis in said living being, wherein said inhibition of the leukemogenesis is realized via the inhibition of BAALC in said living being.

12. The method of claim 11, **characterized in that** said inhibition is realized via knocking out of the gene encoding BAALC (BAALC KO) in said living being.

13. The method of claim 12, **characterized in that** the BAALC KO is realized via the use of proteases, preferably via CRISPR/Cas9 gene-editing.

14. A 'single guide RNA' (sgRNA) molecule targeting the gene encoding BAALC in a living being for use in the prophylaxis and/or treatment of leukemia and/or the development of leukemia.

15. A nucleic acid molecule, preferably the sgRNA molecule of claim 14, comprising any of the nucleotide sequences of SEQ ID NOS:1, 2, 39-78.
